# EUROPÄISCHE PATENTANMELDUNG

(11) **EP 4 306 107 A1**
(43) Veröffentlichungstag der Anmeldung: **17.01.2024**
(21) Anmeldenummer: 22185041.5
(22) Anmeldetag: 14.07.2022
(51) Int. Cl.: A61K 31/194, A61P 25/16, A61P 25/28

(54) **MITTEL ZUR WIEDERHERSTELLUNG DER METALLHOMÖOSTASE**

(71) Anmelder: Oxford Antibiotic Group GmbH, 3430 Tulln an der Donau (AT)
(72) Erfinder: Pretsch, Alexander, 2002 Großmugl (AT); Genov, Miroslav, 3430 Tulln (AT); Pretsch, Dagmar, 2002 Großmugl (AT)
(74) Vertreter: Ellmeyer, Wolfgang

(57) **Zusammenfassung**

Die vorliegende Erfindung Erfindung betrifft Malonsäure und Derivate davon der nachstehenden Formel (I) zur Verwendung als Arzneimittel zur Wiederherstellung der Metallhomöostase bei einem Patienten: 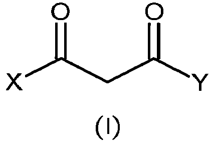 worin X und Y jeweils unabhängig aus -O⁻M⁺, -OR₁ und -NR₂R₃ ausgewählt sind, worin M⁺ aus ein- oder mehrwertigen Metallanionen ausgewählt ist und R₁, R₂ und R₃ jeweils unabhängig aus Wasserstoff und gesättigten, unverzweigten, verzweigten oder zyklischen Kohlenwasserstoff-Resten mit 1 bis 25 Kohlenstoffatomen ausgewählt sind, worin ein oder mehrere Kohlenstoffatome gegebenenfalls durch O oder N ersetzt sind, wobei R₂ und R₃ gegebenenfalls miteinander verbunden sind und zusammen mit dem Stickstoffatom einen heterozyklischen Kohlenwasserstoff-Rest bilden;
sowie eine Verbindung der Formel (I) enthaltende pharmazeutische Zusammensetzungen zur Wiederherstellung der Metallhomöostase.

## Beschreibung

Die vorliegende Erfindung betrifft neue Arzneimittel zur Wiederherstellung der Metallhomöostase, die potenzielle Wirkstoffe gegen neurodegenerative Erkrankungen darstellen.

### STAND DER TECHNIK

Neurologische Erkrankungen wie Alzheimer-, Parkinson-, Huntington- oder Prion-Erkrankungen sind altersabhängige neuronale Erkrankungen, die durch eine Anhäufung von fehlgefalteten Proteinen und den neuronalen Zelltod gekennzeichnet sind. Alle diese Erkrankungen zählen derzeit zu den großen Herausforderungen der Medizin und treten weltweit auf.

Alzheimer liegt mit knapp 50 Millionen Fällen 2020 an der Spitze dieser Erkrankungen. Alle 3 Sekunden entwickelt eine Person auf diesem Planeten Demenz, wovon rund 70 % als Alzheimer bestätigt werden. Generell ist die Alzheimer-Krankheit eine Art von Demenz, die das Denken, Verhalten und Gedächtnis des Patienten beeinflusst. Das Fortschreiten der Krankheit ist moderat und verringert die Neuronen, die mit dem lernenden Teil des Großhirns verbunden sind. Alle gegenwärtigen Ansätze zur Behandlung von Alzheimer bieten nur eine vorübergehende symptomatische Linderung und hemmen oder kehren die zugrunde liegenden Krankheitsmechanismen nicht um, da sie hauptsächlich auf der Grundlage der Amyloidkaskaden-Hypothese entwickelt wurden, die zu keiner zufriedenstellenden Heilung führte.

Als zweite große neurologische Erkrankung unsrer Zeit gilt die Parkinson-Krankheit, eine fortschreitende neurologische Erkrankung, die durch die Degeneration von Dopaminrezeptoren in den Basalganglien verursacht wird. Es ist die am weitesten verbreitete Form des Parkinsonismus, einer Gruppe von Erkrankungen, die durch zwei der vier Hauptzeichen gekennzeichnet sind: Bradykinesie, Rigidität, Zittern und Haltungsinstabilität. Laut der Parkinson Disease Foundation leiden weltweit rund 10 Millionen Menschen an der Parkinson-Krankheit. Somit ist die Parkinson-Krankheit die zweithäufigste neurodegenerative Störung, von der etwa 1,2 % der Weltbevölkerung mit einem Alter von über 70 Jahren betroffen sind. Wie bei Alzheimer gibt es bis heute keine verfügbaren Behandlungen, die in der Lage sind, die Parkinson-Krankheit zu heilen. Das derzeitige Ziel der Therapie besteht daher darin, die Symptome zu lindern. Über 40 Jahre nach seiner Einführung ist Levodopa (L-DOPA, L-3,4-Dihydroxyphenylalanin) der nachstehenden Formel weiterhin die wirksamste Therapie zur Verringerung der mit der Parkinson-Krankheit verbundenen Symptome.

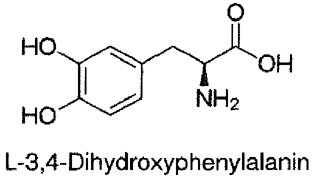

In WO 2017/142855 A1 werden Derivate von Itaconsäure und Malonsäure gemäß den nachstehenden Formeln I bzw. II offenbart: 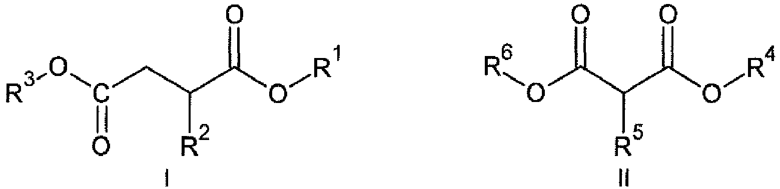 worin R¹ bis R⁶ jeweils unabhängig für Wasserstoff oder gegebenenfalls substituierte Alkyl-, Alkenyl- oder Alkinylgruppen mit einer beliebigen Anzahl an Kohlenstoffatomen stehen, deren optionale Substituenten neben Hydroxy auch eine enorme Bandbreite von mitunter (hetero)aromatischen Kohlenwasserstoffen umfassen können. Von diesen Definitionen sind auch Derivate von Bernsteinsäure und Malein- bzw. Fumarsäure umfasst, wobei Itaconsäure (auch als Methylenbernsteinsäure bezeichnet), Malonsäure, deren Dimethylester, sowie Fumarsäure und 2-Methylfumarsäure (Mesaconsäure) als bevorzugt genannt werden.

Diese Verbindungen sollen als Immunmodulatoren wirken und aufgrund dessen gegen eine mehrseitige Auflistung verschiedenster Arten von Krankheiten einsetzbar sein, worunter sich auch neurodegenerative Erkrankungen wie Alzheimer-, Parkinson-, Huntington- und Prion-Erkrankungen finden lassen. In bevorzugten Ausführungsformen sollen die Verbindungen die Expression verschiedener Proteine hemmen. In den Beispielen wurden allerdings nur die freie Malon- und Itaconsäure auf ihre Wirkung als Hemmer der Succinat-Dehydrogenase untersucht, die auf der strukturellen Ähnlichkeit zu deren Substrat, Bernsteinsäure, beruhen soll. Eine potenzielle Wirksamkeit bei der Behandlung neurodegenerativer Erkrankungen lässt sich daraus nicht ableiten.

Um diesen Mangel an Behandlungsstrategien zu überwinden und andere Therapiemöglichkeiten zu finden, wurde nach neuen Zielen bei der Behandlung dieser Krankheiten geforscht. Bush und Tanzi schlugen 2008 in ihrer "Metallhypothese der Alzheimer-Krankheit" (A. Bush und R. E. Tanzi, "Therapeutics for Alzheimer's disease based on the metal hypothesis", Neurotherapeutics 5(3), 421-432 (2008)) vor, dass ein Zusammenbruch der Metallhomöostase die Hauptursache für neurodegenerative Erkrankungen sei, weswegen Medikamente, die die Metallhomöostase wiederherstellen, viel versprechende neue Therapiestrategien bereitstellen könnten. In der Folge entwickelte die Arbeitsgruppe der vorliegenden Erfinder einen neuen Screening-Assay unter Verwendung von *Caenorhabditis elegans,* eines Fadenwurms aus der Gruppe der Rhabditiden, der auf dieser neuen Strategie der Wiederherstellung der Metallhomöostase durch Hochregulierung der körpereigenen Metallothionein-Biosynthese basiert (siehe Pretsch et al., "Prolongation of metallothionein induction combats Aß and α-synuclein toxicity in aged transgenic Caenorhabditis elegans", Sci. Rep. 10, 11707 (16. Juli 2020)).

Ziel der Erfindung war vor diesem Hintergrund, unter Einsatz des neuen, auf *C. elegans* als Modellorganismus basierenden Assayformats chemische Verbindungen zu ermitteln, die sich als Mittel zur Wiederherstellung der Metallhomöostase eignen und dadurch neue potenzielle Wirkstoffe gegen neurodegenerative Erkrankungen sein könnten.

### ZUSAMMENFASSUNG DER ERFINDUNG

Dieses Ziel erreicht die vorliegende Erfindung durch Bereitstellung von Malonsäure und Derivaten davon der nachstehenden Formel (I) zur Verwendung als Arzneimittel zur Wiederherstellung der Metallhomöostase: 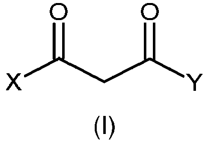 worin X und Y jeweils unabhängig aus -O⁻M⁺, -OR₁ und -NR₂R₃ ausgewählt sind, worin M⁺ aus ein- oder mehrwertigen Metallanionen ausgewählt ist und R₁, R₂ und R₃ jeweils unabhängig aus Wasserstoff und gesättigten, unverzweigten, verzweigten oder zyklischen Kohlenwasserstoff-Resten mit 1 bis 25 Kohlenstoffatomen ausgewählt sind, worin ein oder mehrere Kohlenstoffatome gegebenenfalls durch O oder N ersetzt sind, wobei R₂ und R₃ gegebenenfalls miteinander verbunden sind und zusammen mit dem Stickstoffatom einen heterozyklischen Kohlenwasserstoff-Rest bilden.

Die Erfinder haben nämlich unter Verwendung ihres vor Kurzem entwickelten, oben erwähnten Assayformats überraschenderweise herausgefunden, dass sowohl Malonsäure selbst als auch verschiedenste Derivate der Formel (I), nämlich Ester, Amide und Salze davon, als Mittel zur Wiederherstellung der Metallhomöostase beim Modellorganismus *C. elegans* wirksam sind und daher mit hoher Wahrscheinlichkeit als Wirkstoffe zur Bekämpfung von neurodegenerativen Erkrankungen geeignet sein könnten.

Allerdings wurde diese Wirksamkeit nur für Malonsäure-Derivate bestätigt, in denen die Malonsäure-Gruppierung am zentralen α-Kohlenstoffatom unsubstituiert ist und die keine ungesättigten, insbesondere keine aromatischen, Substituenten aufweisen, wie dies durch die späteren Beispiele und Vergleichsbeispiele belegt wird.

In einer Gruppe von Ausführungsformen, bei denen es sich durchwegs um Salze der Malonsäure handelt, steht in Formel (I) zumindest einer von X und Y für -O⁻M⁺, vorzugsweise stehen allerdings sowohl X als auch Y für -O⁻M⁺, worin M⁺ jeweils unabhängig aus ein- und mehrwertigen Metallanionen ausgewählt ist und M⁺ vorzugsweise für ein Alkalimetall- oder Erdalkalimetallion, noch bevorzugter für ein Alkalimetallion, steht. Als entsprechend wirksame Malonsäuresalze haben sich sowohl solche mit nur einem Carboxylat-Ion als auch solche erwiesen, in denen beide Carboxylgruppen als ionisiertes Carboxylat vorliegen. Bei Letzteren ist allerdings das Herstellungsverfahren etwas einfacher, weswegen diese derzeit erfindungsgemäß zu bevorzugen sind.

Sowohl bei nur einfachen Malonsäuresalzen, in denen nur einer von X und Y für -O⁻M⁺ und der andere für eine Estergruppe -OR₁ oder Amidgruppe -NR₂R₃ steht, als auch bei Diamiden, Diestern und Esteramiden, in denen X und Y beide für -OR₁ oder -NR₂R₃ stehen, sind R₁, R₂ und R₃ vorzugsweise jeweils unabhängig aus Wasserstoff und gesättigten, unverzweigten, verzweigten oder zyklischen Kohlenwasserstoff-Resten mit 1 bis 15 Kohlenstoffatomen, noch bevorzugter aus Wasserstoff und Kohlenwasserstoff-Resten mit 1 bis 10 Kohlenstoffatomen ausgewählt, da ein geringeres Molekulargewicht der Verbindungen die zu verabreichenden Wirkstoffmengen verringert.

In besonders bevorzugten Ausführungsformen sind R₁, R₂ und R₃ jeweils unabhängig aus Wasserstoff, Methyl, Ethyl, Adamantyl und 3-Aminopropyl ausgewählt, da diese vergleichsweise niedrige Molekulargewichte aufweisen und sich in den Testreihen der Erfinder bereits als hochwirksam herausgestellt haben.

Aus letzterem Grund ist die Verbindung gemäß vorliegender Erfindung insbesondere aus den folgenden ausgewählt: Malonsäure (1), Malonsäurediamid (2), Dinatriummalonat (3), Malonsäuredimethylester (4), Malonsäuremethylester-Kaliumsalz (5), Malonsäuremethylesteramid (6), N-(3-Aminopropyl)malonsäureamid (7), Malonsäureadamantan-1-ylester (8), Malonsäureadamantan-1-ylethylester (9), Malonsäureadamantan-1-ylester-N-(3-aminopropyl)amid (10), Malonsäureadamantan-1-ylester-N-(3-aminopropyl)-N-methylamid (11), Malonsäureadamantan-1-ylesterpiperazinamid (12), Malonsäureadamantan-1-ylester-4-(4-aminobutyryl)piperazinamid (13), Malonsäureadamantan-1-ylester-4-aminopiperidinamid (14), Malonsäureadamantan-1-ylester-(4-(4-aminobutyryl)amino)piperidinamid (15), Malonsäuremethylester-N-(adamantan-1-yl)amid (16), N-(Adamantan-1-yl)malonsäureamid (17), N-Adamantan-1-yl-N'-(3-aminopropyl)malonsäurediamid (18), N-Adamantan-1-yl-N'-(3-aminopropyl)-N'-methylmalonsäurediamid (19), N-Adamantan-1-yl-N'-piperazinmalonsäurediamid (20), N-Adamantan-1-yl-N'-(4-(4-aminobutyryl)piperazin)malonsäurediamid (21), N-(3-Aminopropyl)-N'-morpholinmalonsäurediamid (22), N-(3-Aminopropyl)-N'-piperidinmalonsäurediamid (23), N-(3-Aminopropyl)-N'-decahydrochinolinmalonsäurediamid (24), N-(3-Aminopropyl)-N'-decahydroisochinolinmalonsäurediamid (25) und N-(3-Aminopropyl)-N'-(6,6-dimethylbicyclo[3.1.1]heptan-2-ylmethyl)malonsäurediamid (26).

In bevorzugten Ausführungsformen dient die Wiederherstellung der Metallhomöostase zur Behandlung einer neurodegenerativen Erkrankung des Patienten, die besonders bevorzugt aus der Alzheimer-Krankheit, der Parkinson-Krankheit und Chorea Huntington ausgewählt ist und insbesondere die Alzheimer-Krankheit ist, da sie gemäß der oben zitierten "Metallhypothese" am wirksamsten durch die Wiederherstellung der Metallhomöostase behandelbar sein dürfte.

In einem zweiten Aspekt stellt die Erfindung demzufolge auch eine pharmazeutische Zusammensetzung zur Wiederherstellung der Metallhomöostase bei einem Patienten bereit, die eine Verbindung wie oben definiert zur Verwendung gemäß dem ersten Aspekt und zumindest einen pharmazeutisch annehmbaren Exzipienten umfasst und außerdem gegebenenfalls einen oder mehrere weitere pharmazeutisch annehmbare Inhaltsstoffe umfasst, wobei die Zusammensetzung vorzugsweise ebenfalls zur Behandlung einer neurodegenerativen Erkrankung, noch bevorzugter der Alzheimeroder der Parkinson-Krankheit, insbesondere der Alzheimer-Krankheit dient.

### BEISPIELE

Die vorliegende Erfindung wird in der Folge anhand von Beispielen und Vergleichsbeispielen näher beschrieben, die zur Illustration der Erfindung dienen und nicht als einschränkend zu verstehen sind.

Die nachstehend beschriebenen Verbindungen der Beispiele und Vergleichsbeispiele wurden entweder im Handel erstanden oder auf die in den Synthesebeispielen angegebene Weise hergestellt und in der Folge mittels des zuvor erwähnten, in "Pretsch et al." offenbarten Assayformats auf ihre Eignung als Wirkstoffe gegen neurodegenerative Erkrankungen untersucht.

Die Synthesen der Testverbindungen erfolgten anhand von fachüblichen Reaktionsabfolgen, wobei sämtliche Ausgangsprodukte im Handel erhältlich waren oder zuvor aus handelsüblichen Reagenzien synthetisiert wurden, die ohne weitere Reinigung in den Synthesen eingesetzt wurden. Die Charakterisierungen erfolgten anhand von ¹H-NMR-Spektren, die unter Verwendung eines Avance AV400 Spektrometers von Bruker bei 400 MHz aufgenommen wurden, sowie von mit einem LCMS-8040 von Shimadzu aufgenommenen Massenspektren.

Konkret wurden in der vorliegenden ersten Testreihe die nachstehenden Verbindungen auf ihre Wirksamkeit untersucht. Weitere Tests sind derzeit Gegenstand der Forschungsarbeiten der Erfinder.

Als Beispiele für die vorliegende Erfindung wurden die folgenden getestet:

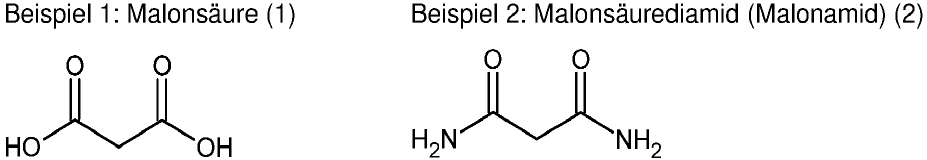

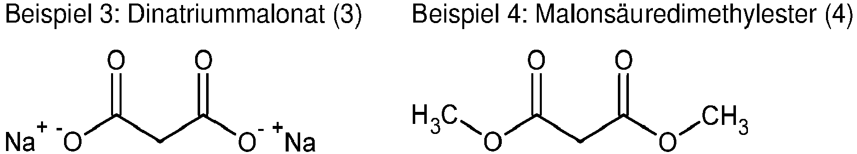

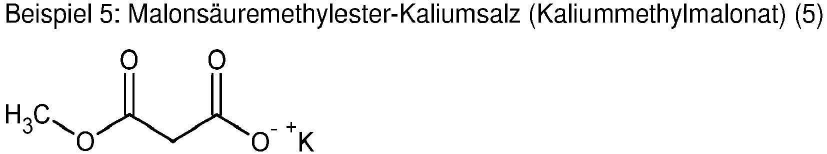

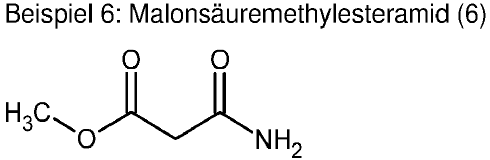

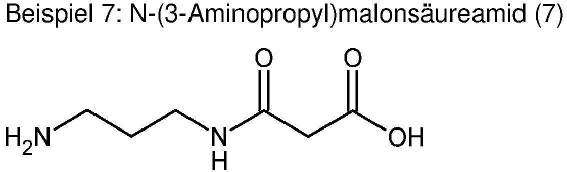

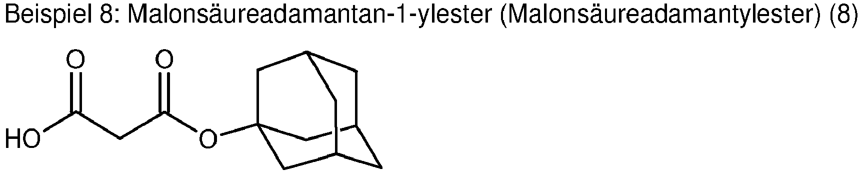

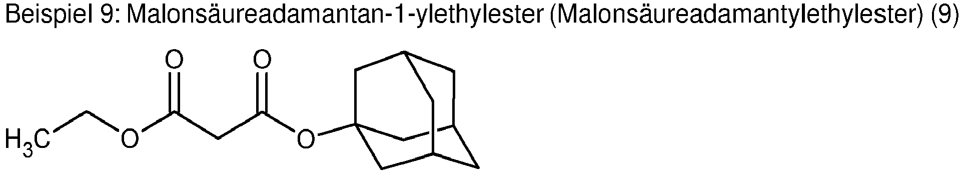

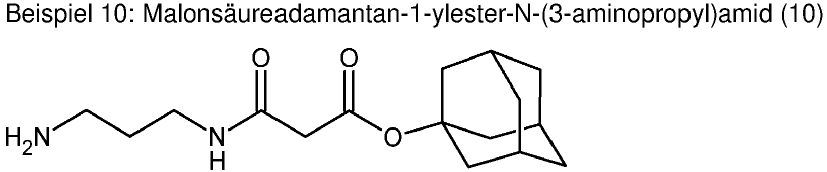

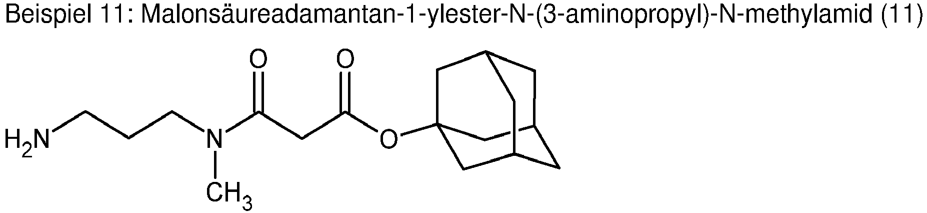

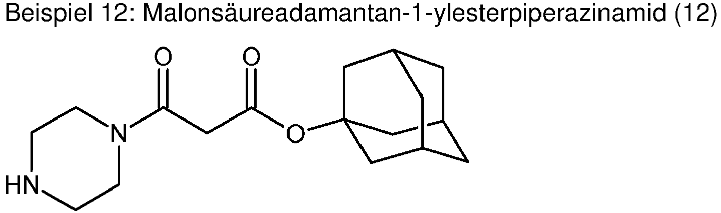

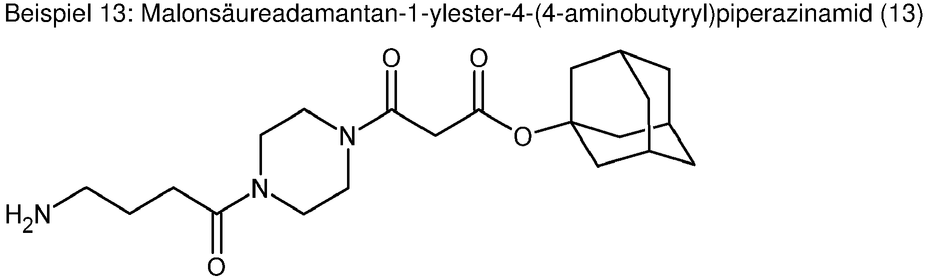

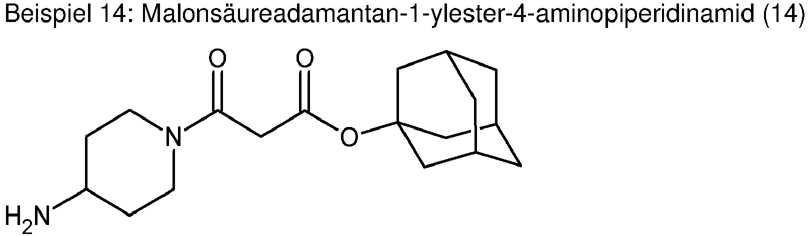

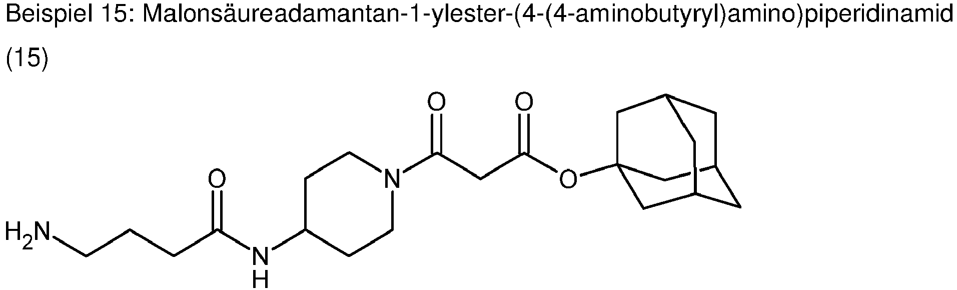

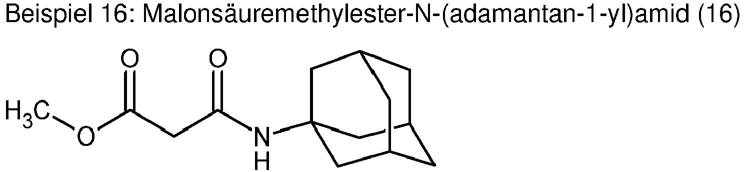

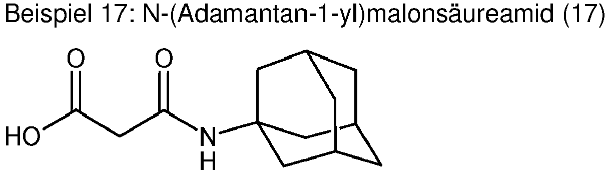

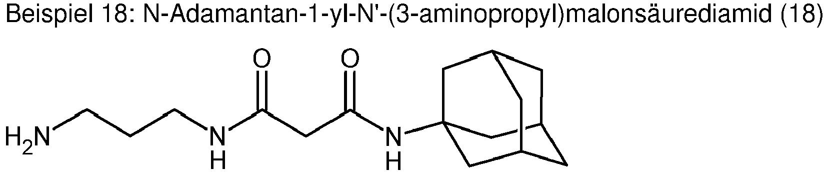

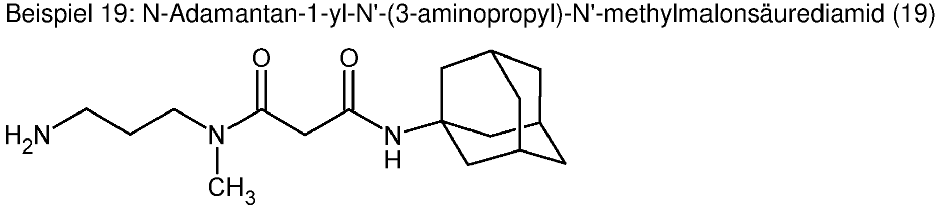

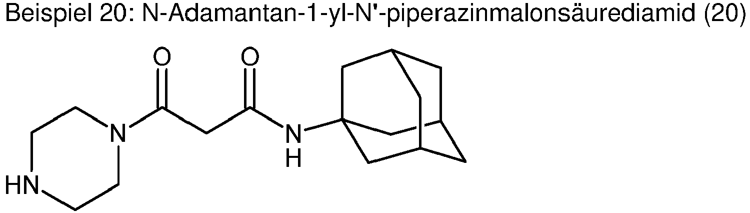

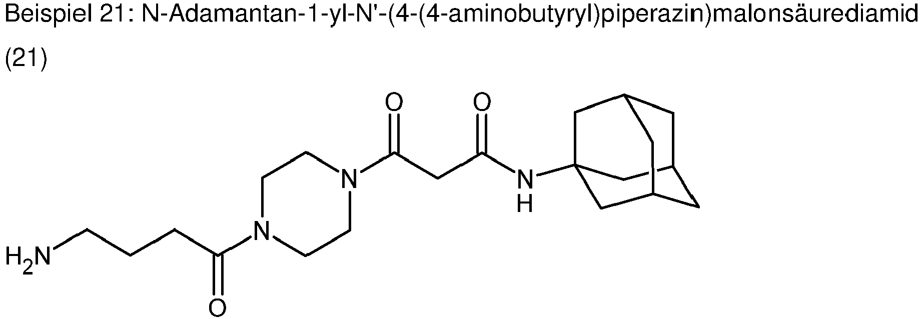

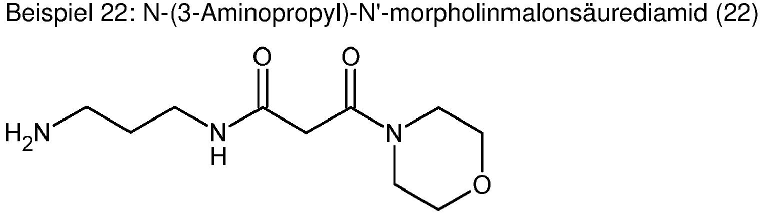

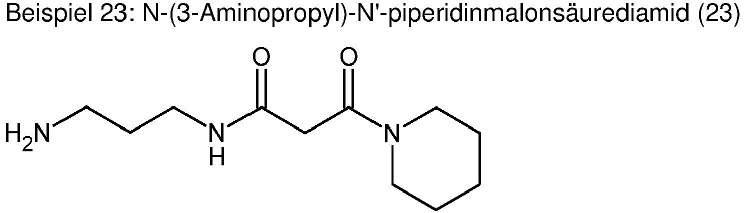

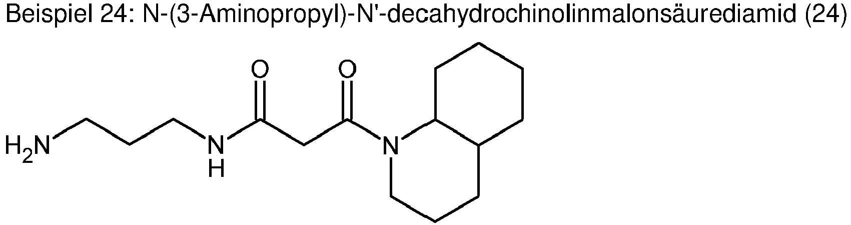

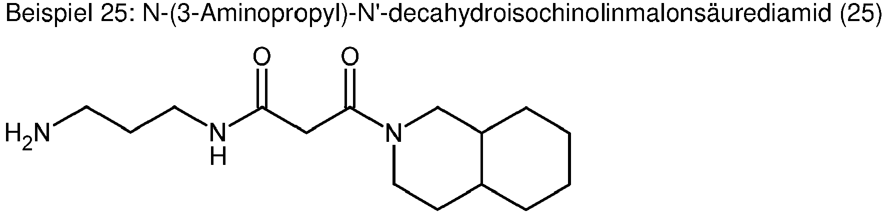

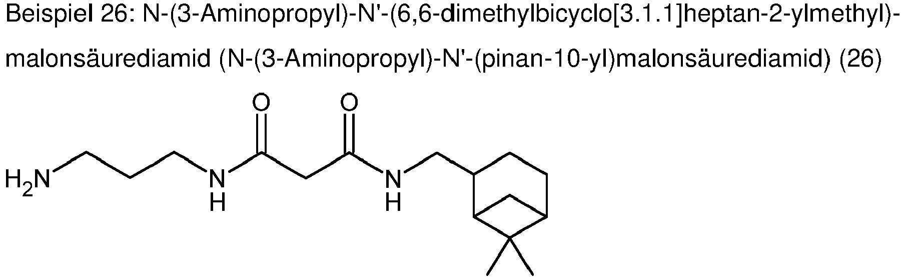

Und als Vergleichsbeispiele wurden die folgenden Verbindungen untersucht:

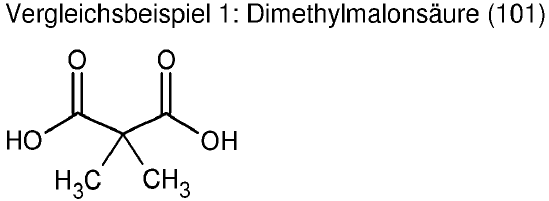

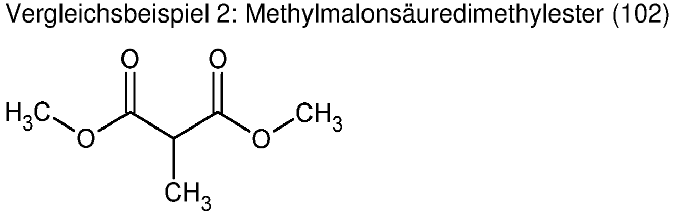

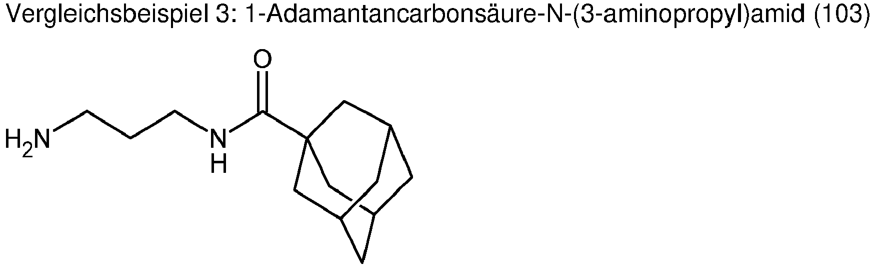

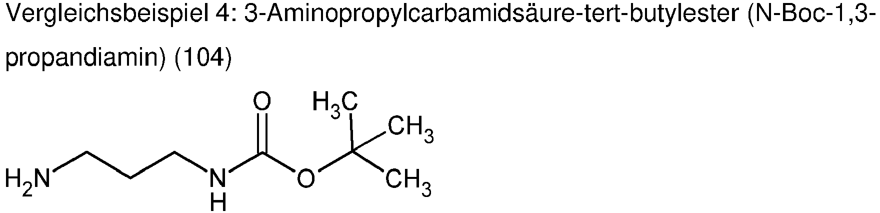

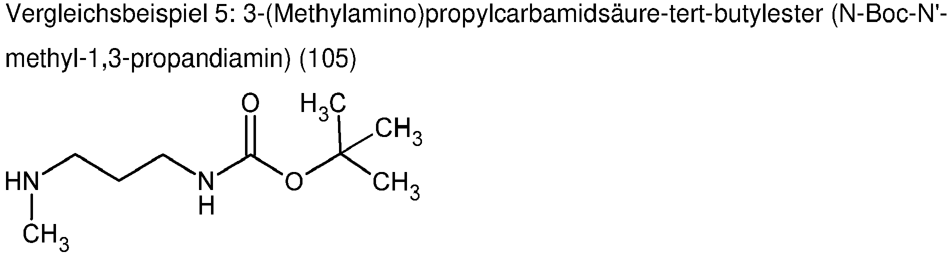

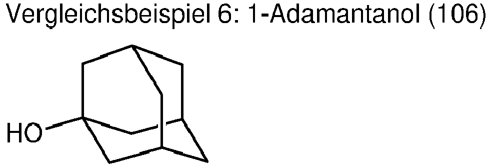

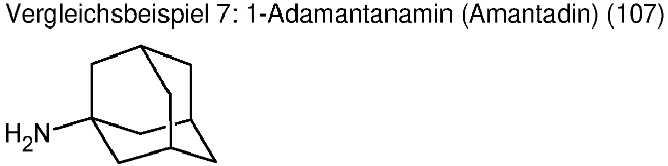

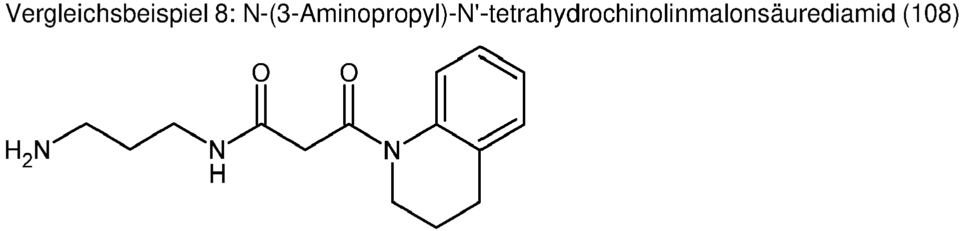

Davon wurden die Verbindungen (1) bis (7) und (101) bis (107) aus kommerziellen Quellen bezogen und die übrigen Verbindungen (8) bis (26) und (108) wie nachstehend beschrieben hergestellt.

### Svnthesebeispiel 1

Herstellung von Malonsäureadamantan-1-ylester (8)

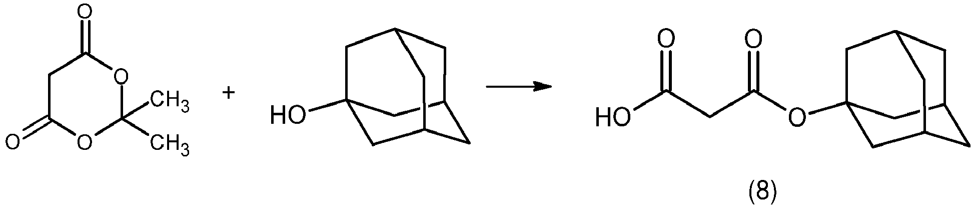

Eine Lösung von 1,0 g (6,57 mmol) Adamantan-1-ol und 0,95 g (6,57 mmol) 2,2-Dimethyl-1,3-dioxan-4,6-dion in Toluol wurde 12 h lang rückflusserhitzt, wonach das Reaktionsgemisch auf Raumtemperatur abkühlen gelassen wurde. Anschließend wurden 60 ml gesättigte wässrige NaHCO₃-Lösung langsam zugesetzt, was zu einer Phasentrennung führte. Die Toluolphase wurde verworfen, und die wässrige Phase wurde mit 100 ml 1 N HCl vorsichtig angesäuert. Die saure Lösung wurde mit Dichlormethan extrahiert, und die organische Phase wurde über MgSO₄ getrocknet, filtriert und bis zur Trockene eingedampft, wobei 1,5 g der Titelverbindung als farblose Kristalle erhalten wurden.

¹H-NMR (CD₃OD): δ = 1,71 (m, 6H, Adamantyl), 2,10-2,20 (m, 9H, Adamantyl), 3,25 (s, 2H), 4,81 (brs, 1H). MS (ESI-): m/z (%) 237 (100, [M-H]⁻), 283 (47, [M+HCOO⁻]).

### Synthesebeispiel 2

### Herstellung von Malonsäureadamantan-1-ylethylester (9)

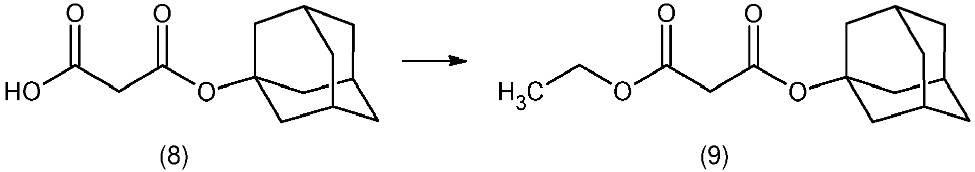

Zu einer Lösung von 35 mg Malonsäureadamantan-1-ylester (8) in 10 ml Ethanol wurden 2 ml 4 N HCl in Dioxan zugesetzt. Nach 24 h Rühren bei Raumtemperatur wurde das Reaktionsgemisch eingeengt und der Rückstand mittels Säulenchromatographie (Cyclohexan/Ethylacetat 95:5) gereinigt, was die Titelverbindung als farblose Flüssigkeit ergab (35 mg; % d. Th.).

¹H-NMR (CDCl₃): δ = 1,25 (t, 3H, J=7,14 Hz), 1,62 (m, 6H), 2,08-2,13 (m, 9H), 3,23 (s, 2H), 4,16 (q, 2H, J=7,14 Hz). MS (ESI+): m/z (%) 267 (13, [M+H]⁺), 284 (6, [M+H₂O]⁺), 289 (100, [M+Na]⁺).

### Svnthesebeispiel 3

### Herstellung von Malonsäureadamantan-1-ylester-N-(3-aminopropyl)amid (10)

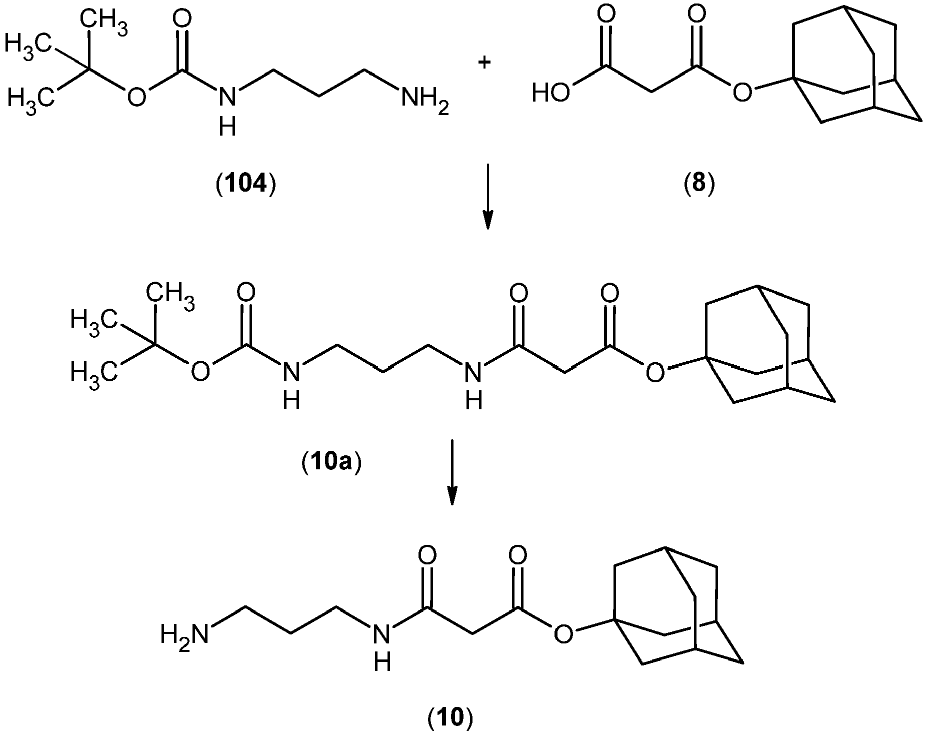

Zu einer auf 0 °C gekühlten Lösung von 22 mg (0,126 mmol) 3-Aminopropylcarbamidsäure-tert-butylester (N-Boc-1,3-propandiamin) (104), 30 mg (0,126 mmol) Malonsäureadamantan-1-ylester (8) und 45 µl (0,252 mmol) Diisopropylethylamin (DIPEA) in 8 ml Dimethylformamid (DMF) wurden 54 mg (0,126 mmol) (1-Cyano-2-ethoxy-2-oxo-ethylidenaminooxy)dimethylaminomorpholinocarbenium-hexafluorophosphat (COMU) zugesetzt. Nach 2 h Rühren unter Erwärmenlassen auf Raumtemperatur wurde das Reaktionsgemisch mit Ethylacetat verdünnt, mit 1 N HCl und gesättigter wässriger NaHCO₃-Lösung gewaschen, über MgSO₄ getrocknet, filtriert und bis zur Trockene eingedampft. Der das Boc-geschützte Malonsäureadamantan-1-ylester-N-(3-aminopropyl)amid (10a) enthaltende Rückstand (47 mg) wurde ohne weitere Reinigung in der nächsten Stufe eingesetzt.

MS (ESI+): m/z (%): 395 (100, [M+H]⁺), 417 (100, [M+Na]⁺), 811 (100, [2M+Na]⁺).

Der so erhaltene Rückstand wurde in 8 ml Dichlormethan gelöst, 1,26 ml 4 N HCl in Dioxan wurden zugesetzt, und das Gemisch wurde 2 h lang bei Raumtemperatur gerührt. Anschließend wurden die flüchtigen Bestandteile im Vakuum entfernt, wodurch das Hydrochlorid der Titelverbindung (10) in Form eines gelben Öls (34 mg) erhalten wurde.

¹H-NMR (CDCl₃): δ = 1,60-1,72 (m, 10H), 2,10-2,18 (m, 9H), 2,85 (s, 2H), 3,22-3,25 (m, 1H), 3,62-3,70 (m, 2H), 3,75-3,79 (m, 1H). MS (ESI+): m/z (%) 295 (100, [M+H]⁺), 589 (80, [2M+H]⁺), 611 (15, [2M+Na]⁺).

### Synthesebeispiel 4

### Herstellung von Malonsäureadamantan-1-ylester-N-(3-aminopropyl)-N-methylamid (11)

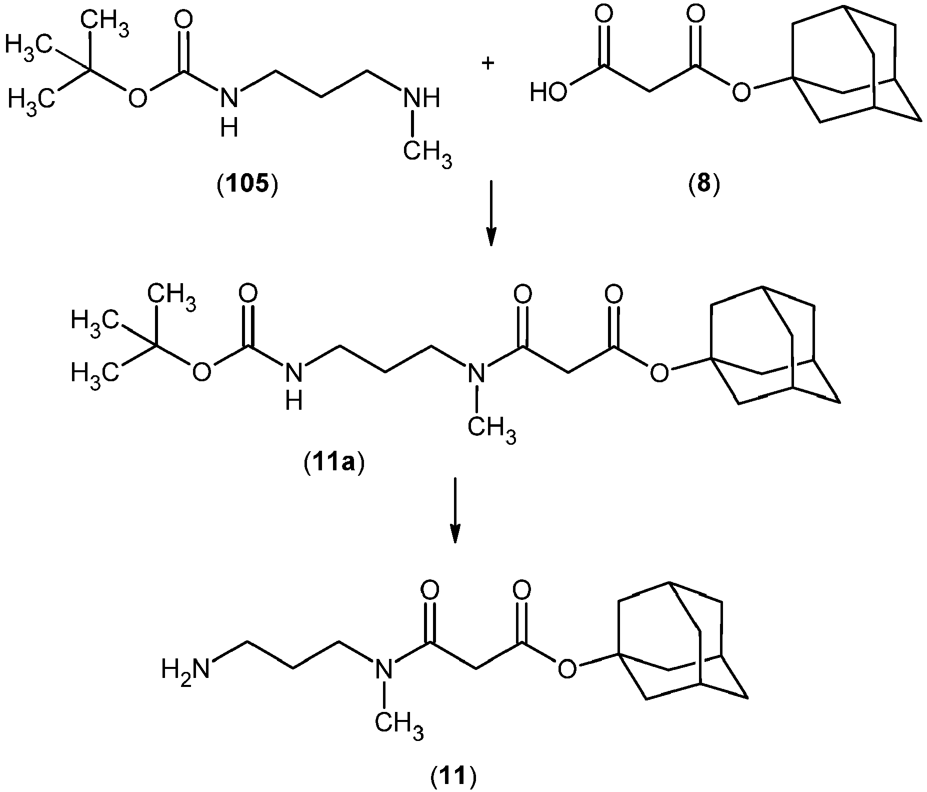

Zu einer Lösung von Malonsäureadamantylester (8) (289 mg, 1,211 mmol), 3-(Methylamino)propylcarbamidsäure-tert-butylester (105) (228 mg, 1,211 mmol) und Triethylamin (TEA) (340 µl, 2,422 mmol) in DMF (6 ml) wurden 1 ml einer 1,6 M Lösung von Propanphosphonsäureanhydrid (T3P) in Ethylacetat langsam zugesetzt und 1 h lang bei Raumtemperatur gerührt. Anschließend wurde das Reaktionsgemisch zur Hydrolyse mit gesättigter Kochsalzlösung versetzt und mit Dichlormethan extrahiert. Der Extrakt wurde über MgSO₄ getrocknet, filtriert und bis zur Trockene eingedampft. Der Rückstand wurde mittels Säulenchromatographie (Dichlormethan/Ethylacetat 70:30) gereinigt, wobei das Boc-geschützte Malonsäureadamantan-1-ylester-N-(3-aminopropyl)-N-methylamid (11a) in Form eines farblosen Öls (370 mg) erhalten wurde.

MS (ESI+) m/z (%): 409 (100, [M+H]⁺, 431 (100, [M+Na]⁺), 309 (100, [M-Boc]⁺), 839 (100, [2M+Na]⁺).

Das so erhaltene Produkt (0,96 mmol) wurde in 20 ml Dichlormethan gelöst, 9,6 ml 4 N HCl in Dioxan wurden zugesetzt, und das Gemisch wurde 1,5 h lang bei Raumtemperatur gerührt. Anschließend wurden die flüchtigen Bestandteile im Vakuum entfernt, wodurch das Hydrochlorid der Titelverbindung (11) in Form eines gelben Öls (260 mg) erhalten wurde.

¹H-NMR (DMSO-d₆): δ = 1,63-1,75 (m, 10H), 2,10-2,20 (m, 9H), 2,85 (s, 2H), 2,97 (s, 3H) 3,22-3,25 (m, 1H), 3,61-3,72 (m, 2H), 3,73-3,82 (m, 1H). MS (ESI+) m/z (%): 309 (100, [M+H]⁺), 331 (17, [M+Na]⁺), 617 (77, [2M]⁺).

### Svnthesebeispiel 5

### Herstellung von Malonsäureadamantan-1-ylesterpiperazinamid (12)

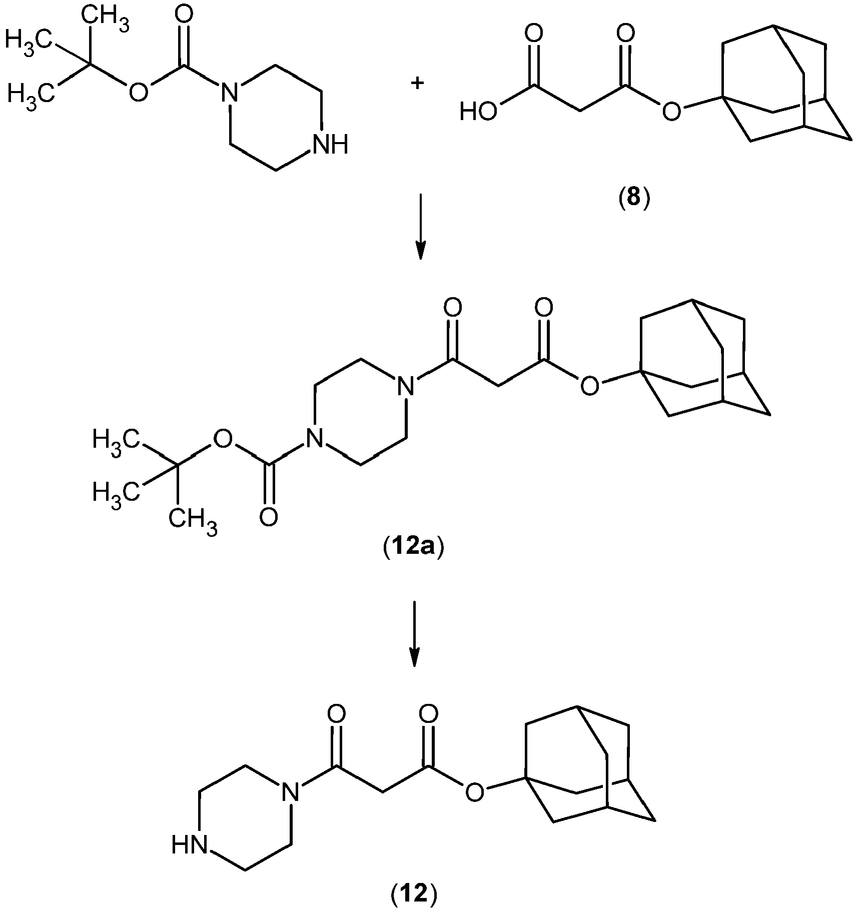

Die Synthese erfolgte analog zu Synthesebeispiel 4 aus Malonsäureadamantylester (8) (150 mg, 0,630 mmol), 1-Piperazincarbamidsäure-tert-butylester (117 mg, 0,630 mmol) und TEA (176 µl, 1,26 mmol) in DMF (7 ml) mit 512 µl einer 1,6 M Lösung von T3P in Ethylacetat. Reinigung des Rückstands mittels Säulenchromatographie (Dichlormethan/Ethylacetat 90:10→50:50) ergab die Boc-geschützte Titelverbindung (12a) in Form eines weißen Feststoffs (232 mg).

MS (ESI+) m/z (%): 407 (100, [M+H]⁺), 429 (100, [M+Na]⁺), 836 (100, [2M+Na]⁺).

Das so erhaltene Produkt (0,571 mmol) wurde analog zu Synthesebeispiel 4 entschützt, wobei das Hydrochlorid der Titelverbindung (12) in Form eines weißen Feststoffs (169 mg) erhalten wurde.

¹H-NMR (DMSO-d₆): δ = 1,6 (t, 6H, J=3,0 Hz), 2,03 (d, 6H, J=3,0 Hz), 2,11 (brs, 3H), 3,05 (dt, 4H, J=22,6, 5,2 Hz), 3,32 (brs, 1H), 3,48 (s, 2H), 3,63 (dt, 4H, J=22,6, 5,2 Hz) MS (ESI+) m/z (%): 307 (100, [M+H]⁺), 329 (25, [M+Na]⁺), 635 (15, [2M+Na]⁺).

### Svnthesebeispiel 6

### Herstellung von Malonsäureadamantan-1-ylester-4-(4-aminobutyryl)piperazinamid (13)

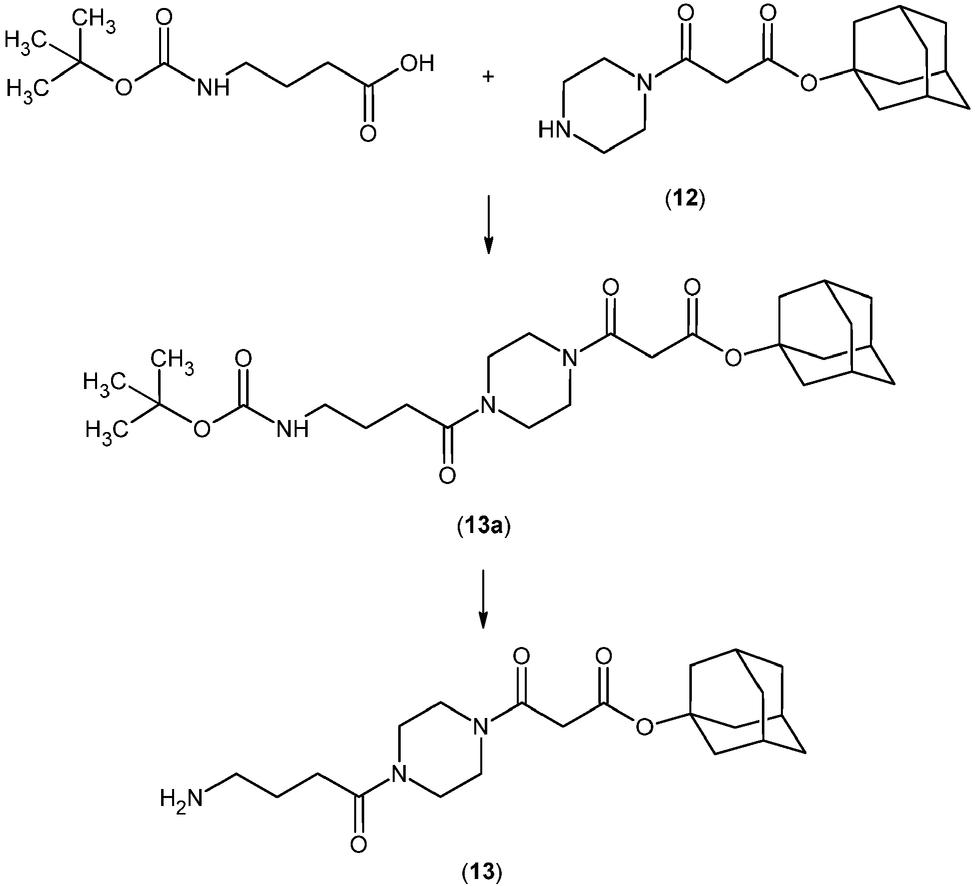

Die Synthese erfolgte analog zu Synthesebeispiel 4 aus Malonsäureadamantan-1-ylesterpiperazinamid (12) (180 mg, 0,525 mmol), 4-(tert-Butoxycarbonylamino)buttersäure (107 mg, 0,525 mmol) und Triethylamin (TEA) (219 µl, 1,575 mmol) in DMF (7 ml) mit 427 µl einer 1,6 M Lösung von T3P in Ethylacetat. Reinigung mittels Säulenchromatographie (Dichlormethan/Ethylacetat 80:20) ergab die Boc-geschützte Titelverbindung (13a) in Form eines weißen Feststoffs (190 mg).

MS (ESI+) m/z (%): 492 (100, [M+H]⁺), 514 (100, [M+Na]⁺), 392 (72, {M+H-Boc]⁺).

Das so erhaltene Produkt (0,386 mmol) wurde analog zu Synthesebeispiel 4 entschützt, wobei das Hydrochlorid der Titelverbindung (12) in Form eines weißen Pulvers (151 mg) erhalten wurde.

¹H-NMR (DMSO-d₆): 1,59 (brs, 6H), 1,76 (quint, 2H, J=7,4 Hz), 2,03 (d, 6H, J=3Hz), 2,10 (brs, 3H), 2,45 (quint, 2H, J=7,4 Hz), 2,74-2,83 (m, 2H), 3,34 (brs, 2H), 3,40-3,45 (m, 8H), 3,54 (s, 2H). MS (ESI+) m/z (%): 392 (100, [M+H]⁺), 414 (100, [M+Na]⁺), 430 (10, [M+K]⁺), 805 (69, [2M+Na]⁺).

### Svnthesebeispiel 7

### Herstellung von Malonsäureadamantan-1-ylester-4-aminopiperidinamid (14)

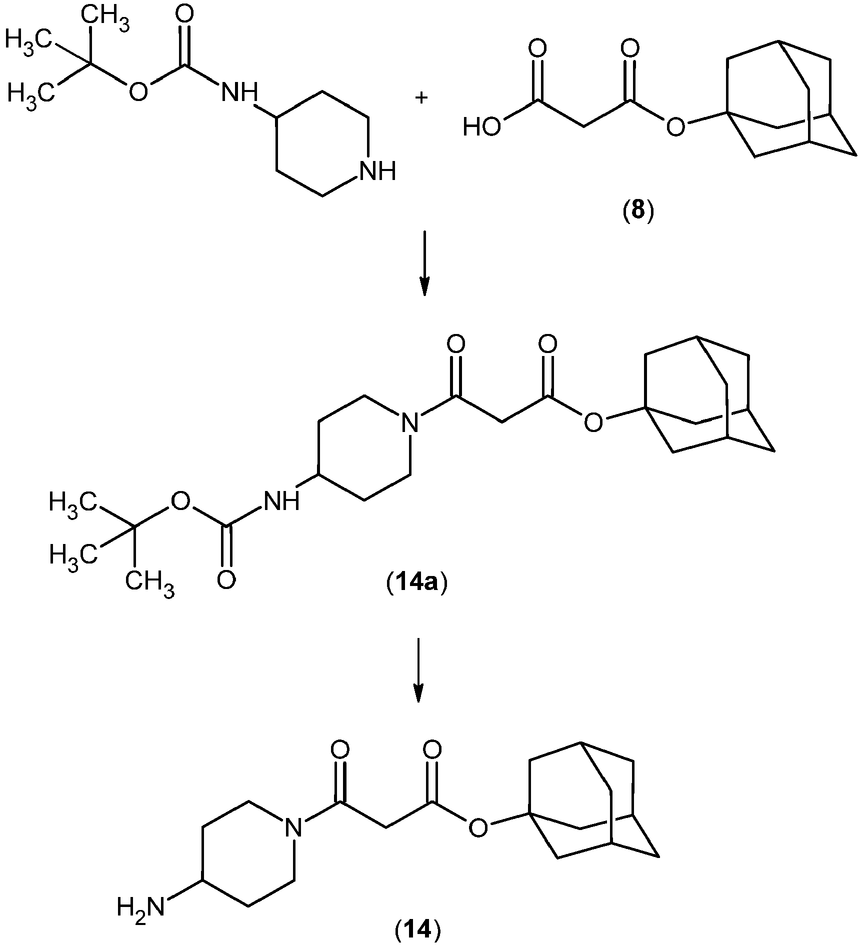

Die Synthese erfolgte analog zu Synthesebeispiel 4 aus Malonsäureadamantan-1-ylester (8) (150 mg, 0,630 mmol), Piperidin-4-ylcarbamidsäure-tert-butylester (126 mg, 0,630 mmol) und Triethylamin (TEA) (176 µl, 1,26 mmol) in DMF (7 ml) mit 512 µl einer 1,6 M Lösung von T3P in Ethylacetat. Reinigung mittels Säulenchromatographie (Dichlormethan/Ethylacetat 90:10→80:20) ergab die Boc-geschützte Titelverbindung (14a) in Form eines weißen Feststoffs (236 mg).

MS (ESI+) m/z (%): 421 (100, [M+H]⁺), 443 (100, [M+Na]⁺), 864 (100, [2M+Na]⁺).

Das so erhaltene Produkt (0,561 mmol) wurde analog zu Synthesebeispiel 4 entschützt, wobei das Hydrochlorid der Titelverbindung (14) in Form eines weißen Pulvers (175 mg) erhalten wurde.

MS (ESI+) m/z (%): 321 (100, [M+H]⁺), 343 (7, [M+Na]⁺), 641 (24, [2M+H]⁺), 663 (95, [2M+Na]⁺).

### Synthesebeispiel 8

Herstellung von Malonsäureadamantan-1-ylester-(4-(4-aminobutyryl)amino)piperidinamid (15)

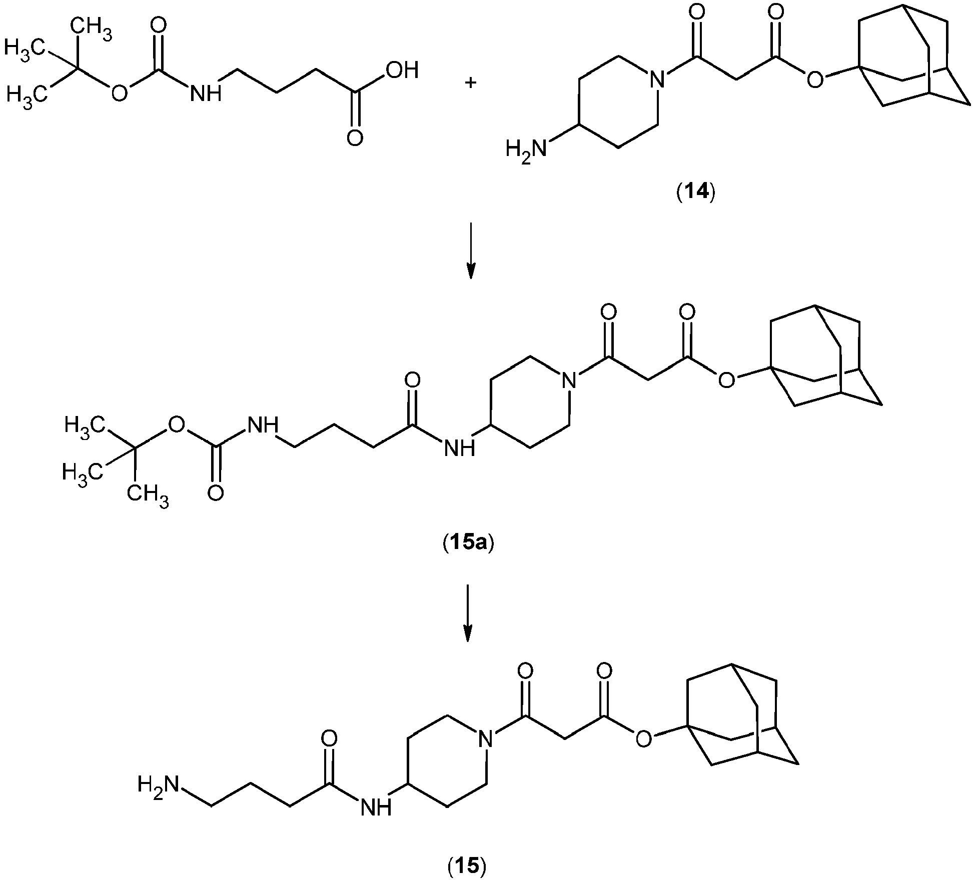

Die Synthese erfolgte analog zu Synthesebeispiel 4 aus Malonsäureadamantan-1-yl-ester-4-aminopiperidinamid (14) (140 mg, 0,392 mmol), 4-(tert-Butoxycarbonylamino)-buttersäure (80 mg, 0,392 mmol) und Triethylamin (TEA) (164 µl, 1,176 mmol) in DMF (7 ml) mit 319 µl einer 1,6 M Lösung von T3P in Ethylacetat. Reinigung mittels Säulenchromatographie (Dichlormethan/Ethylacetat 80:20→Ethylacetat) ergab die Boc-geschützte Titelverbindung (15a) in Form eines gelben Öls (83 mg).

MS (ESI+) m/z (%): 506 (100, [M+H]⁺), 528 (100, [M+Na]⁺), 406 (78, [M-Boc+H]⁺).

Das so erhaltene Produkt (0,164 mmol) wurde analog zu Synthesebeispiel 4 entschützt, wobei das Hydrochlorid der Titelverbindung (14) in Form eines gelben Pulvers (80 mg) erhalten wurde.

¹H-NMR (DMSO-d₆): δ = 1,64 (dt, 3H, J=13,1, 2,8 Hz), 1,66 (dt, 3H, J=13,1, 2,8 Hz), 1,74 (sept, 3H, J=2,8 Hz), 1,80-1,87 (m, 6H), 2,09-2,13 (m, 6H), 2,16 (t, 2H, J=7,4 Hz), 2,65 (t, 2H, J=7,7 Hz), 3,04-3,13 (m, 2H), 3,35 (ddd, 2H, J=14,7, 6,8, 2,8 Hz), 3,55 (s, 2H), 4,11-4,21 (m, 1H), 7,97 (brs, 2H). MS (ESI⁺) m/z (%): 406 (100, [M+H]⁺), 428 (100, [M+Na]⁺), 811 (100, [2M+H]⁺), 833 (53, [2M+Na]⁺).

### Svnthesebeispiel 9

### Herstellung von Malonsäuremethylester-N-(adamantan-1-yl)amid (16)

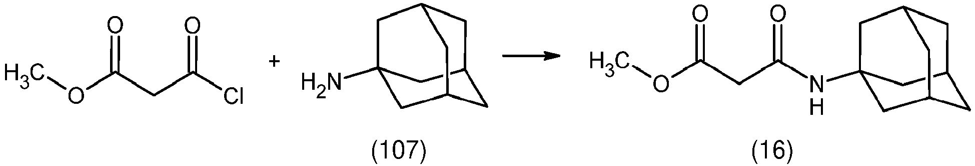

Malonsäuremethylesterchlorid (322 µl, 3,005 mmol) wurde unter Inertgasatmosphäre in 30 ml Dichlormethan gelöst und auf -20 °C abgekühlt, wonach mit einer Spritze eine Lösung von 1-Adamantanamin (107) (1,0 g, 6,612 mmol) in 10 ml Dichlormethan langsam zugesetzt wurde. Das Reaktionsgemisch wurde bei Raumtemperatur 1 h lang gerührt, mit gesättigter wässriger NH₄CI-Lösung gequencht und mit Dichlormethan extrahiert. Die organische Phase wurde über MgSO₄ getrocknet, filtriert und bis zur Trockene eingedampft, wobei die Titelverbindung (760 mg) in Form von weißen Kristallen erhalten wurde.

¹H-NMR (CDCl₃): δ = 1,68 (m, 6H), 2,01-2,10 (m, 9H), 3,22 (s, 2H), 3,74 (s, 3H), 6,64 (brs, 1H). MS (ESI+): m/z (%) 252 (100, [M+H]⁺), 274 (100, [M+Na]⁺), 503 (78, [2M+H]⁺).

### Svnthesebeispiel 10

### Herstellung von N-(Adamantan-1-yl)malonsäureamid (17)

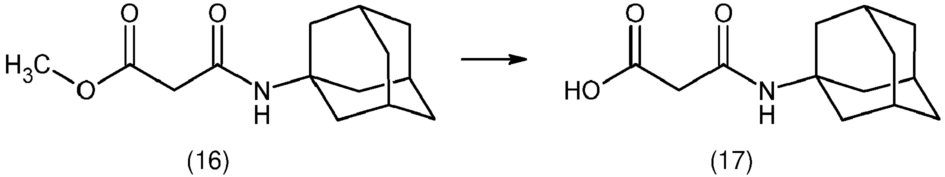

Zu einer Lösung von 750 mg (2,98 mmol) Malonsäuremethylester-N-(adamantan-1-yl)amid (16) in 30 ml THF/H₂O (2:1) wurden 6 ml 1 M wässrige NaOH zugesetzt und bei Raumtemperatur gerührt. Nach 1 h wurde das THF am Rotationsverdampfer entfernt, und die verbliebene wässrige Lösung mit 2 N HCl angesäuert und mit Ethylacetat extrahiert. Die organische Phase wurde über MgSO₄ getrocknet, filtriert und bis zur Trockene eingedampft, wobei die Titelverbindung als cremefarbenes Pulver erhalten wurde (670 mg).

MS (ESI-): m/z (%) 236 (100, [M-H]⁻), 473 (100, [2M-H]⁻).

### Svnthesebeispiel 11

### Herstellung von N-Adamantan-1-yl-N'-(3-aminopropyl)malonsäurediamid (18)

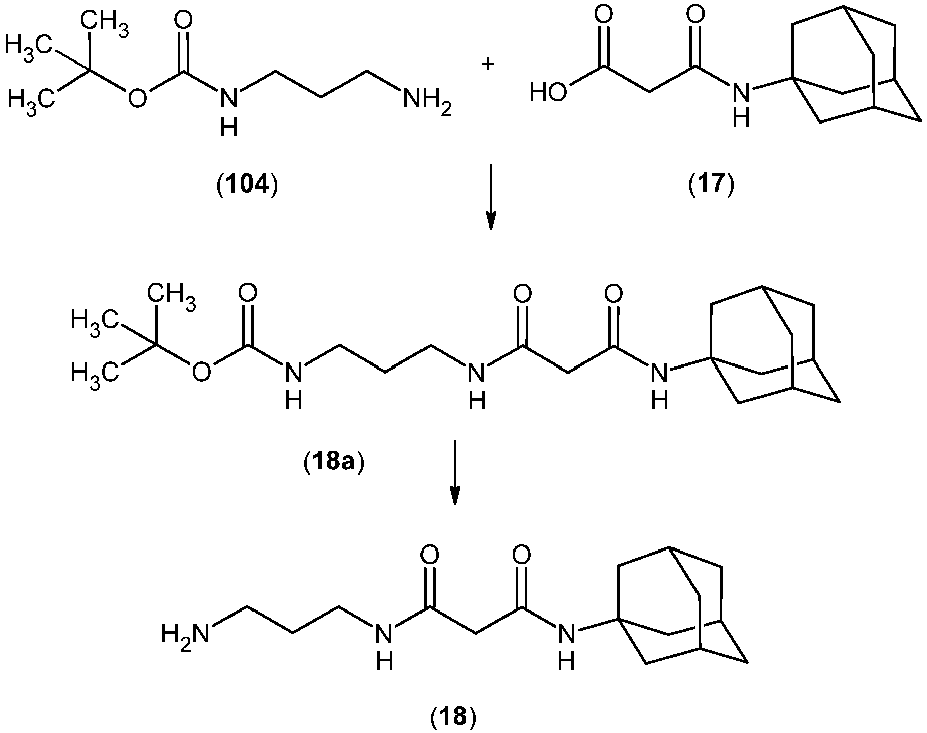

Zu einer auf 0 °C gekühlten Lösung von N-(Adamantan-1-yl)malonsäureamid (17) (50 mg, 0,211 mmol), 3-Aminopropylcarbamidsäure-tert-butylester (104) (37 mg, 0,211 mmol) und 4-Dimethylaminopyridin (DMAP) (13 mg, 0,106 mmol) in Dichlormethan (8 ml) wurde Diisopropylcarbodiimid (DIC) (40 µl, 0,253 mmol) zugesetzt. Nach 4 h Rühren unter Erwärmenlassen auf Raumtemperatur wurde das Reaktionsgemisch bis zur Trockene eingedampft. Der das Boc-geschützte N-Adamantan-1-yl-N'-(3-aminopropyl)malonsäurediamid (18a) enthaltende Rückstand (59 mg, 0,202 mmol) wurde ohne weitere Reinigung in der nächsten Stufe eingesetzt.

MS (ESI+): m/z (%) 394 (100, [M+H]⁺), 415 (100, [M+Na]⁺), 294 (70, [M-BOC+H]⁺), 787 (40, [2M+H]⁺).

Der so erhaltene Rückstand wurde in 6 ml Dichlormethan gelöst, 2 ml 4 N HCl in Dioxan wurden zugesetzt, und das Gemisch wurde 2 h lang bei Raumtemperatur gerührt. Anschließend wurden die flüchtigen Bestandteile im Vakuum entfernt, wodurch das Hydrochlorid der Titelverbindung (18) in Form eines gelben Öls (55 mg) erhalten wurde.

¹H-NMR (DMSO-d₆): δ = 1,61 (m, 6H, Adamantyl), 1,68 (quint, 2H, J=7,1 Hz), 1,90-2,00 (m, 9H, Adamantyl), 2,74-2,82 (m, 2H), 2,98 (s, 2H), 3,12 (q, 2H, J=6,42 Hz), 7,58 (s, 1H). MS (ESI+): m/z (%) 294 (90, [M+H]⁺), 316 (100, [M+Na]⁺), 608 (45, [2M+Na]⁺).

### Synthesebeispiel 12

### Herstellung von N-Adamantan-1-yl-N'-(3-aminopropyl)-N'-methylmalonsäurediamid (19)

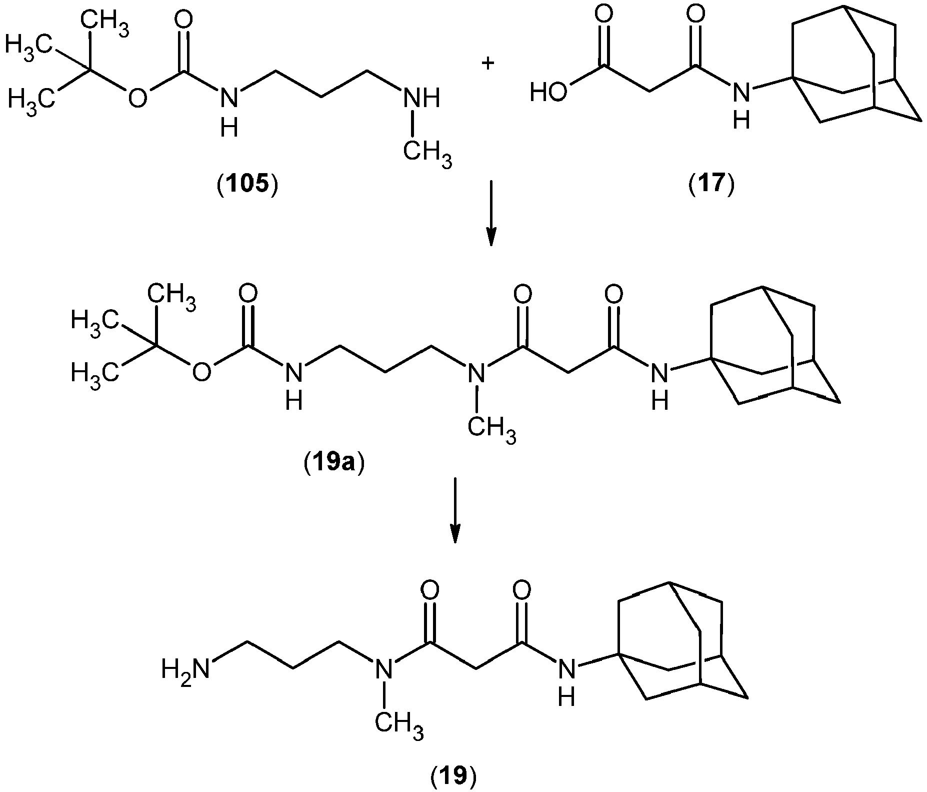

Die Synthese erfolgte analog zu Synthesebeispiel 4 aus N-(Adamantan-1-yl)malon-säureamid (17) (50 mg, 0,211 mmol), 3-(Methylamino)propylcarbamidsäure-tert-butylester (105) (40 mg, 0,211 mmol) und Triethylamin (TEA) (60 µl, 0,422 mmol) in DMF (10 ml) mit 175 µl einer 1,6 M Lösung von T3P in Ethylacetat. Reinigung mittels Säulenchromatographie (Dichlormethan/Ethylacetat 70:30) ergab die Boc-geschützte Titelverbindung (19a) in Form eines gelben Öls (83 mg).

MS (ESI+): m/z (%) 408 (100, [M+H]⁺), 430 (100, [M+Na]⁺), 308 (100, [M-BOC+H]⁺), 815 (50, [2M+H]⁺).

Das so erhaltene Produkt (46 mg, 0,113 mmol) wurde analog zu Synthesebeispiel 4 entschützt, wobei das Hydrochlorid der Titelverbindung (19) in Form eines gelben Öls (33 mg) erhalten wurde.

¹H-NMR (DMSO-d₆): δ = 1,61 (m, 6H, Adamantyl), 1,74 (quint, 2H, J=5,5 Hz), 1,97-2,07 (m, 9H, Adamantyl), 2,75-2,84 (m, 2H), 2,93 (s, 3H), 3,26 (s, 2H), 3,35 (t, 2H, J=5,5 Hz), 3,41 (brs, 2H), 7,50 (s, 1H). MS (ESI+): m/z (%) 308 (100, [M+H]⁺), 330 (33, [M+Na]⁺), 615 (100, [2M+H]⁺).

### Svnthesebeispiel 13

### Herstellung von N-Adamantan-1-yl-N'-piperazinmalonsäurediamid (20)

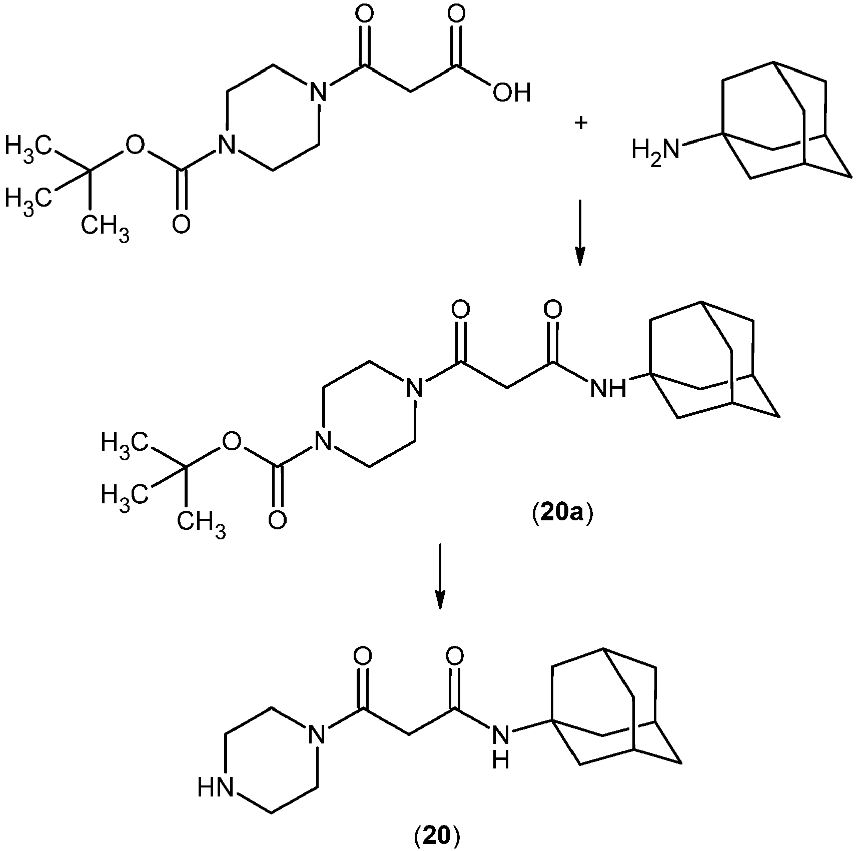

Die Synthese erfolgte analog zu Synthesebeispiel 4 aus handelsüblichem Malonsäure-4-(tert-butoxycarbonyl)piperidinamid (250 mg, 0,918 mmol), 1-Adamantanamin (107) (139 mg, 0,918 mmol) und Triethylamin (TEA) (265 µl, 1,84 mmol) in DMF (17 ml) mit 750 µl einer 1,6 M Lösung von T3P in Ethylacetat. Reinigung mittels Säulenchromatographie (Dichlormethan/Ethylacetat 90:10→50:50) ergab die Boc-geschützte Titelverbindung (20a) in Form eines weißen Feststoffs (260 mg).

¹H-NMR (CDCl₃): δ = 1,46 (s, 9H), 1,67 (t, 7H, J=3,9 Hz), 1,98 (d, 6H, J=3,1 Hz), 2,06 (brs, 3H), 3,24 (s, 2H), 3,40-3,46 (m, 4H), 3,52-3,55 (m, 2H), 3,58-3,61 (m, 2H), 6,73 (brs, 1H). MS (ESI+) m/z (%): 406 (100, [M+H]⁺), 428 (100, [M+Na]⁺), 833 (2M+Na]⁺).

Das so erhaltene Produkt (0,647 mmol) wurde analog zu Synthesebeispiel 4 entschützt, wobei das Hydrochlorid der Titelverbindung (20) in Form eines weißen Pulvers (192 mg) erhalten wurde.

¹H-NMR (DMSO-d₆): 1,61 (brs, 6H), 1,9 (d, 6H, J=2,95 Hz), 2,00 (brs, 3H), 3,00 (brs, 2H), 3,10 (brs, 2H), 3,66-3,68 (m, 4H), 4,01 (brs, 2H). MS (ESI+) m/z (%): 306 (100, [M+H]⁺), 329 (7, [M+Na]⁺), 633 (95, [2M+Na]⁺).

### Svnthesebeispiel 14

### Herstellung von N-Adamantan-1-yl-N'-(4-(4-aminobutyryl)piperazin)malonsäurediamid (21)

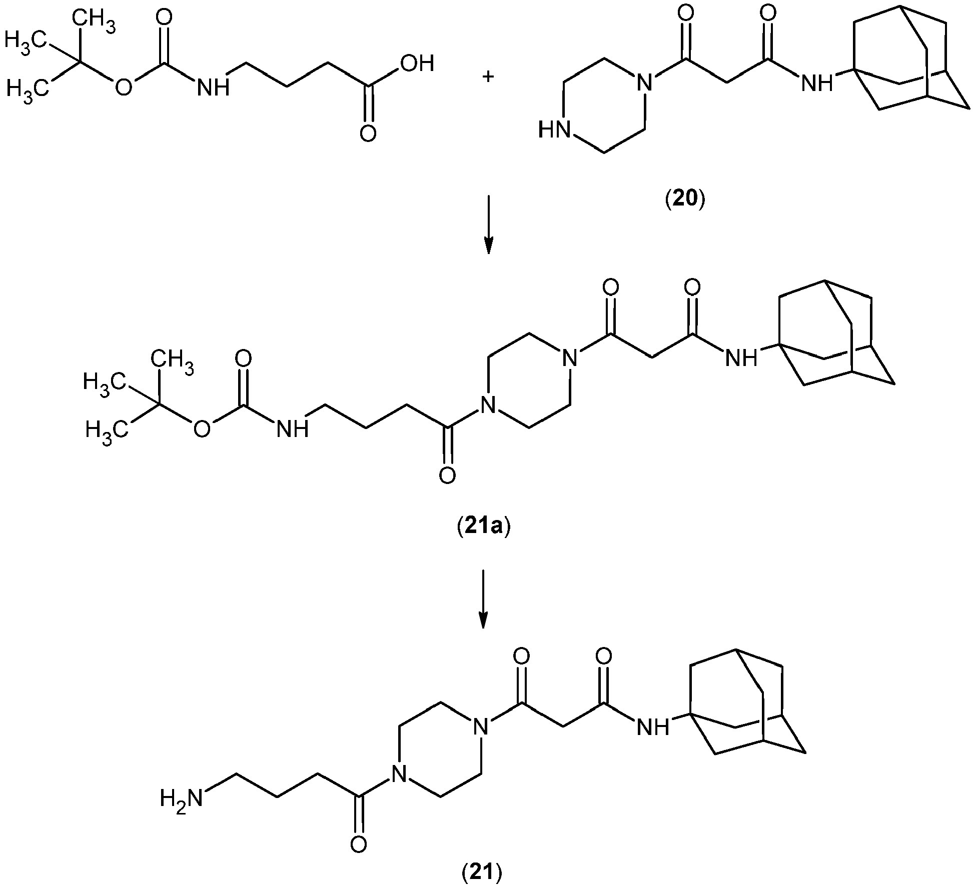

Die Synthese erfolgte analog zu Synthesebeispiel 4 aus dem Hydrochlorid von N-Adamantan-1-yl-N'-piperazinmalonsäurediamid (20) (140 mg, 0,410 mmol), 4-(tert-Butoxycarbonylamino)buttersäure (83 mg, 0,410 mmol) und Triethylamin (TEA) (171 µl, 1,23 mmol) in DMF (7 ml) mit 333 µl einer 1,6 M Lösung von T3P in Ethylacetat. Reinigung mittels Säulenchromatographie (Ethylacetat/Methanol 95:5) ergab die Boc-geschützte Titelverbindung (21a) in Form eines weißen Feststoffs (180 mg).

MS (ESI+) m/z (%): 491 (100, [M+H]⁺), 513 (100, [M+Na]⁺), 391 (38, [M-Boc+H]⁺).

Das so erhaltene Produkt (0,367 mmol) wurde analog zu Synthesebeispiel 4 entschützt, wobei das Hydrochlorid der Titelverbindung (21) in Form eines weißen Pulvers (150 mg) erhalten wurde.

¹H-NMR (DMSO-d₆): 1,59 (brs, 6H), 1,77 (quint, 2H, J=6,4 Hz), 1,89 (brs, 6H), 1,98 (brs, 3H), 2,43-2,47 (m, 2H), 2,76-2,81 (m, 2H), 3,26-3,30 (m, 2H), 3,39-3,45 (m, 8H), 4,34 (brs, 2H), 7,61 (s, 1H).

### Svnthesebeispiel 15

### Herstellung von N-(3-Aminopropyl)-N'-morpholinmalonsäurediamid (22)

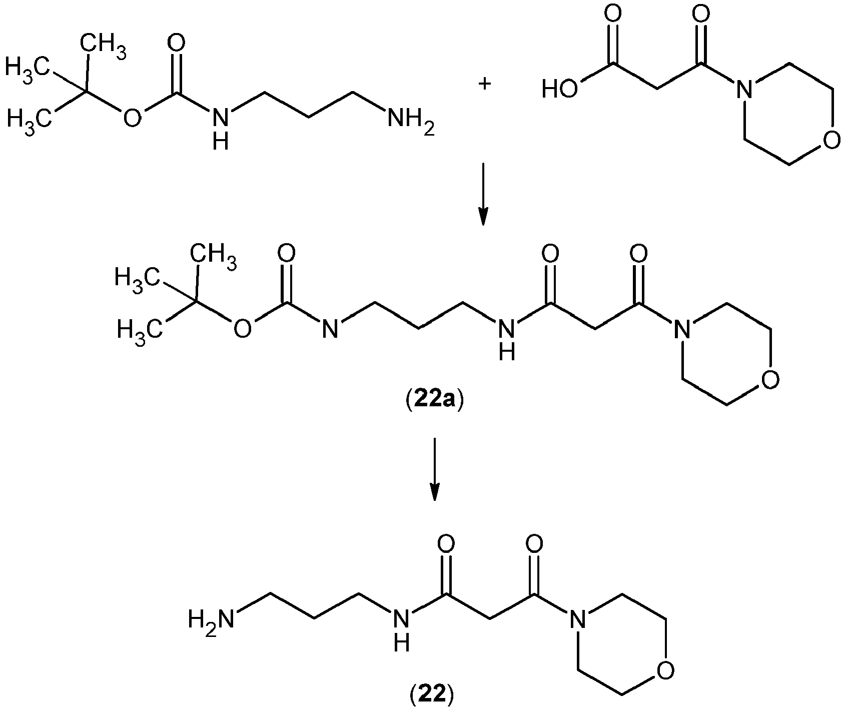

Die Synthese erfolgte analog zu Synthesebeispiel 4 aus handelsüblichem N-Morpholinmalonsäureamid (90 mg, 0,520 mmol), 3-Aminopropylcarbamidsäure-tert-butylester (N-Boc-1,3-propandiamin) (104) (91 mg, 0,520 mmol) und Triethylamin (TEA) (145 µl, 1,04 mmol) in DMF (5 ml) mit 423 µl einer 1,6 M Lösung von T3P in Ethylacetat. Reinigung mittels Säulenchromatographie (Ethylacetat/Methanol 95:5) ergab die Boc-geschützte Titelverbindung (22a) in Form eines gelben Öls (90 mg).

MS (ESI+) m/z (%): 330 (100, [M+H]⁺), 352 (100, [M+Na]⁺), 230 (100, [M-Boc+H]⁺), 681 (100, [2M+Na]⁺).

Das so erhaltene Produkt (0,273 mmol) wurde analog zu Synthesebeispiel 4 entschützt, wobei das Hydrochlorid der Titelverbindung (22) in Form eines gelblichen Öls (76 mg) erhalten wurde.

MS (ESI+) m/z (%): 230 (100, [M+H]⁺), 252 (100, [M+Na]⁺), 459 (11, [2M+H]⁺), 481 (100, [2M+Na]⁺).

### Synthesebeispiel 16

### Herstellung von N-(3-Aminopropyl)-N'-piperidinmalonsäurediamid (23)

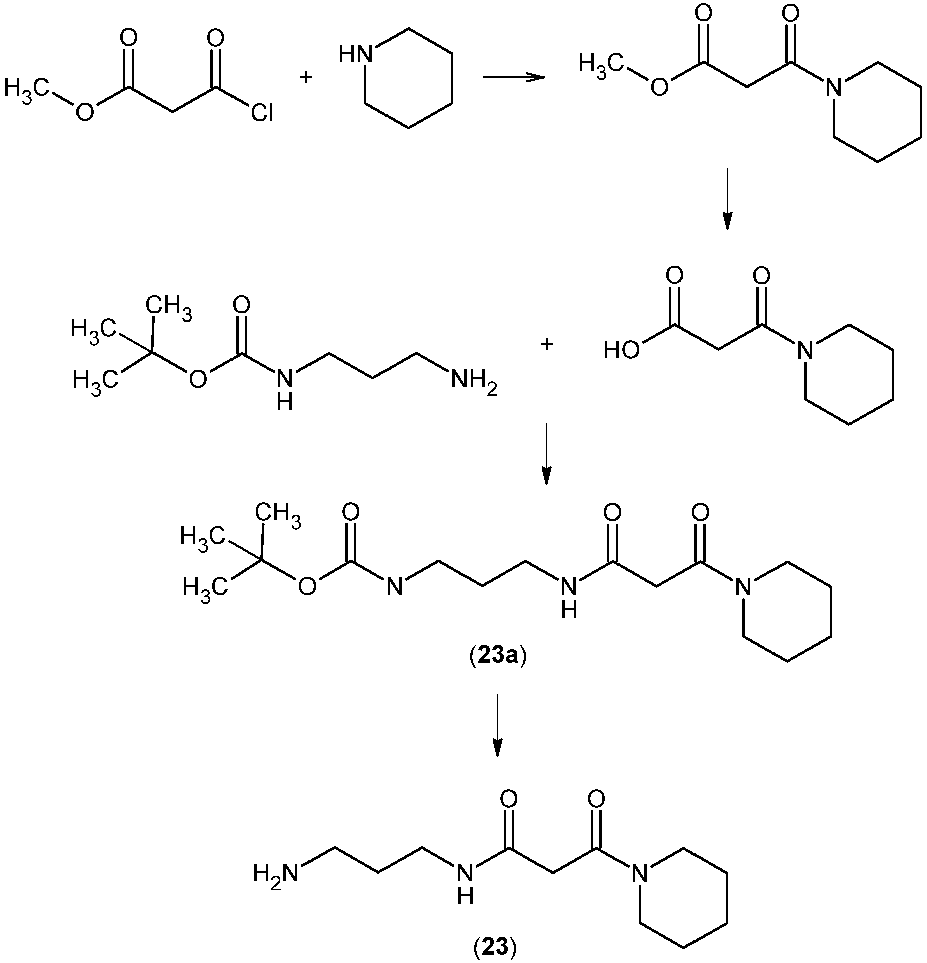

Die Synthese erfolgte analog zu einer Kombination der Synthesebeispiele 9, 10 und 4, wobei zunächst analog zu Synthesebeispiel 9 aus Malonsäuremethylesterchlorid (250 µl, 2,33 mmol) und Piperidin (506 µl, 5,126 mmol) bei -20 °C in 10 ml Dichlormethan das Malonsäuremethylester-N-piperidinamid hergestellt wurde, das danach analog zu Synthesebeispiel 10 in THF/H₂O (2:1) mit wässriger NaOH zum Malonsäure-N-piperidinamid hydrolysiert wurde.

MS (ESI+) m/z (%): 172 (100, [M+H]⁺), 194 (50, [M+H]⁺).

Dieses (280 mg, 1,636 mmol) wurde danach analog zu Synthesebeispiel 4 mit 3-Aminopropylcarbamidsäure-tert-butylester (N-Boc-1,3-propandiamin) (285 mg, 1,636 mmol) und TEA (456 µl, 3,272 mmol) in DMF (6 ml) mit 1,33 ml einer 1,6 M Lösung von T3P in Ethylacetat umgesetzt. Reinigung mittels Säulenchromatographie (Dichlormethan/Ethylacetat 50:50→Ethylacetat) ergab die Boc-geschützte Titelverbindung (23a) in Form eines farblosen Öls (370 mg).

¹H-NMR (CDCl₃): δ = 1,43 (s, 9H), 1,53-1,60 (m, 4H), 1,63-1,68 (m, 5H), 3,14 (q, 2H, J=6,5 Hz), 3,31 (s, 2H), 3,33 (q, 2H, J=6,5 Hz), 3,45 (t, 2H, J=4,8 Hz), 3,56 (t, 2H, J=4,8 Hz), 7,78 (s, 1H). MS (ESI+) m/z (%): 328 (100, [M+H]⁺), 350 (100, [M+Na]⁺), 228 (80, [M-Boc+H]⁺), 677 (100, [2M+Na]⁺).

Das so erhaltene Produkt (1,13 mmol) wurde analog zu Synthesebeispiel 4 entschützt, wobei das Hydrochlorid der Titelverbindung (23) in Form eines weißen Pulvers (330 mg) erhalten wurde.

¹H-NMR (DMSO-d₆): δ = 1,37-1,43 (m, 2H), 1,45-1,50 (m, 2H), 1,53-1,59 (m, 2H), 1,69 (quint, 2H, J=8,3 Hz), 2,75-2,83 (m, 2H), 3,12 (q, 2H, 4,8 Hz), 3,35-3,41 (m, 4H), 7,96 (s, 1H). MS (ESI+) m/z (%): 228 (100, [M+H]⁺), 250 (16, [M+Na]⁺), 455 (40, [2M+H]⁺).

### Svnthesebeispiel 17

### Herstellung von N-(3-Aminopropyl)-N'-decahydrochinolinmalonsäurediamid (24)

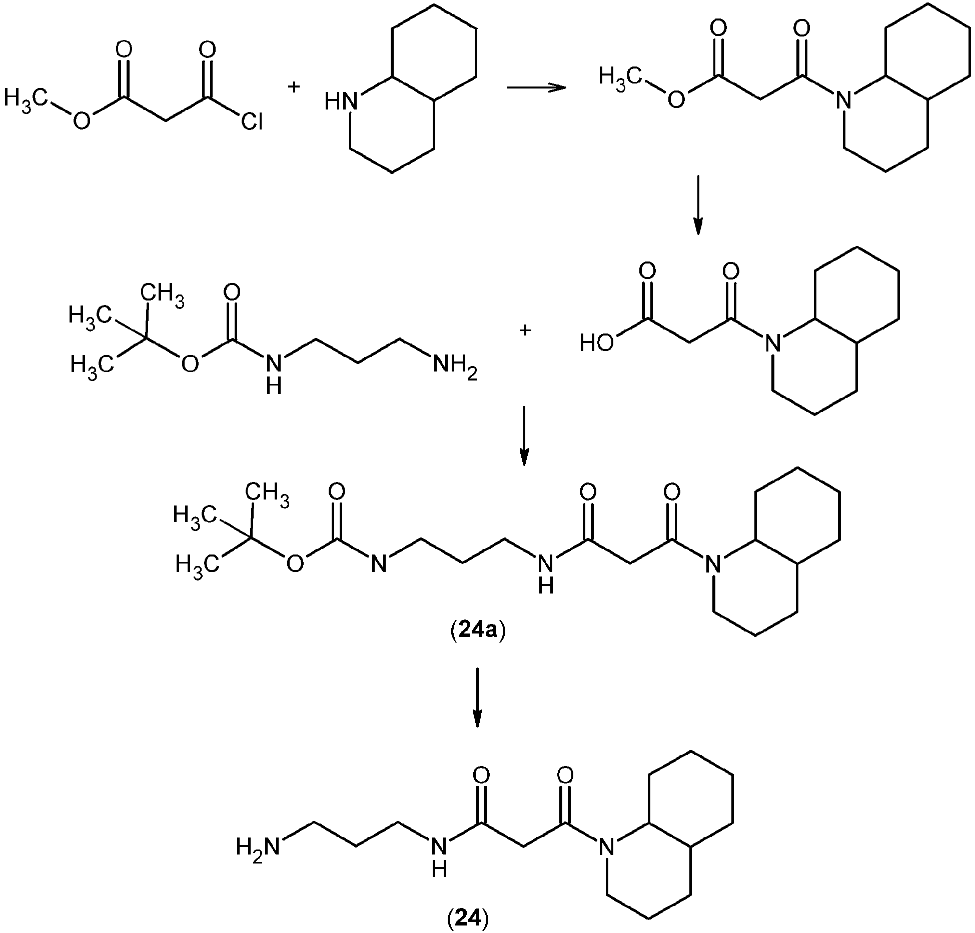

Die Synthese erfolgte analog zu Synthesebeispiel 16 aus Malonsäuremethylesterchlorid (175 µl, 1,63 mmol) und trans-Decahydrochinolin (500 mg, 3,59 mmol) zum Malonsäuremethylester-N-decahydrochinolinamid, das zum Malonsäure-N-decahydrochinolinamid hydrolysiert wurde.

MS (ESI+) m/z (%): 226 (100, [M+H]⁺), 248 (85, [M+Na]⁺).

Dieses (190 mg, 0,843 mmol) wurde mit 3-Aminopropylcarbamidsäure-tert-butylester (N-Boc-1,3-propandiamin) (147 mg, 0,843 mmol) und TEA (236 µl, 1,686 mmol) in DMF (6 ml) mit 685 µl einer 1,6 M Lösung von T3P in Ethylacetat umgesetzt. Reinigung mittels Säulenchromatographie (Dichlormethan/Ethylacetat 50:50→40:60) ergab die Boc-geschützte Titelverbindung (24a) in Form eines farblosen Öls (230 mg).

MS (ESI+) m/z (%): 382 (100, [M+H]⁺), 404 (80, [M+Na]⁺), 282 (37, [M-Boc+H]⁺), 785 (15, [2M+Na]⁺).

Das so erhaltene Produkt (0,603 mmol) wurde auf analoge Weise entschützt, wobei das Hydrochlorid der Titelverbindung (24) in Form eines weißen Pulvers (215 mg) erhalten wurde.

MS (ESI+) m/z (%): 282 (100, [M+H)⁺], 304 (30, [M+Na]⁺), 563 (80, [2M+H]⁺), 585 (60, [2M+Na]⁺).

### Svnthesebeispiel 18

### Herstellung von N-(3-Aminopropyl)-N'-decahydroisochinolinmalonsäurediamid (25)

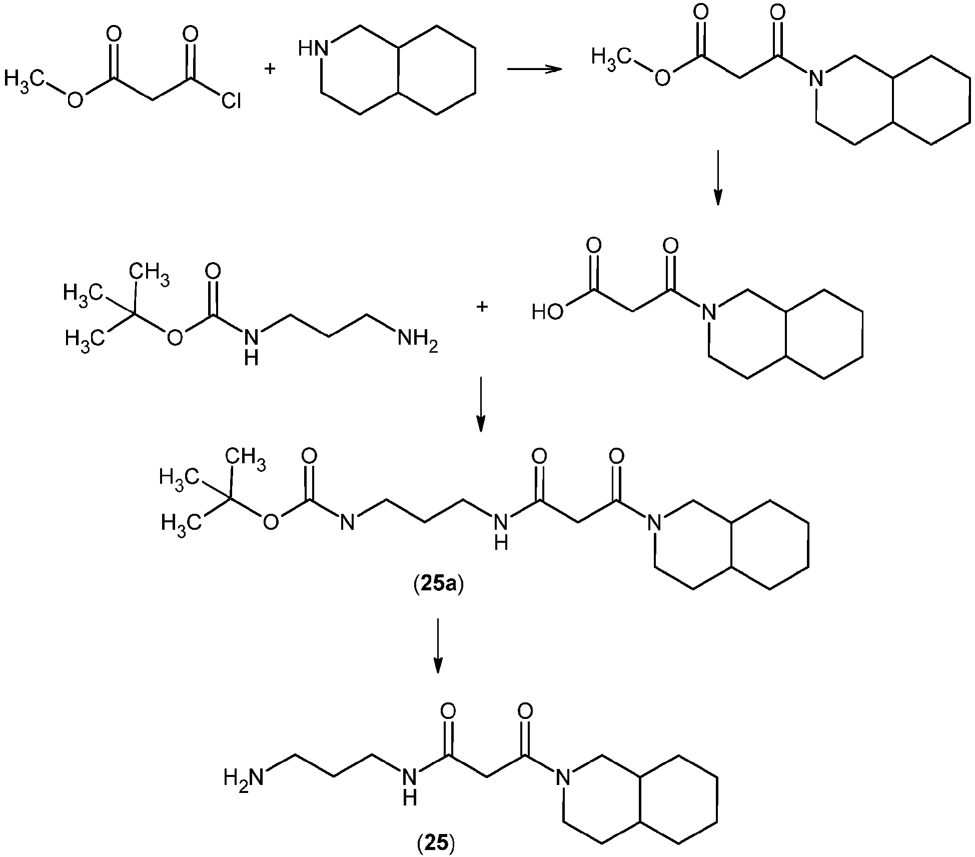

Die Synthese erfolgte analog zu Synthesebeispiel 17 aus Malonsäuremethylesterchlorid (175 µl, 1,63 mmol) und Decahydroisochinolin (500 mg, 3,59 mmol) zum Malonsäuremethylester-N-decahydroisochinolinamid, das zum Malonsäure-N-decahydroisochinolinamid hydrolysiert wurde.

MS (ESI+) m/z (%): 226 (100, [M+H]⁺), 248 (100, [M+Na]⁺), 473 (36, [2M+Na]⁺).

Dieses (272 mg, 1,207 mmol) wurde mit 3-Aminopropylcarbamidsäure-tert-butylester (N-Boc-1,3-propandiamin) (210 mg, 1,207 mmol) und TEA (336 µl, 2,414 mmol) in DMF (10 ml) mit 980 µl einer 1,6 M Lösung von T3P in Ethylacetat umgesetzt. Reinigung mittels Säulenchromatographie (Ethylacetat) ergab die Boc-geschützte Titelverbindung (25a) in Form eines farblosen Öls (306 mg).

MS (ESI+) m/z (%): 382 (100, [M+H]⁺), 404 (100, [M+Na]⁺), 282 (100, [M-Boc+H]⁺), 785 (100, [2M+Na]⁺).

Das so erhaltene Produkt (0,786 mmol) wurde auf analoge Weise entschützt, wobei das Hydrochlorid der Titelverbindung (25) in Form eines weißen Pulvers (280 mg) erhalten wurde.

MS (ESI+) m/z (%): 282 (100, [M+H]⁺), 304 (20, [M+Na]⁺), 563 (40, [2M+H]⁺), 585 (50, [2M+Na]⁺).

### Svnthesebeispiel 19

### Herstellung von N-(3-Aminopropyl)-N'-(pinan-10-yl)malonsäurediamid (26)

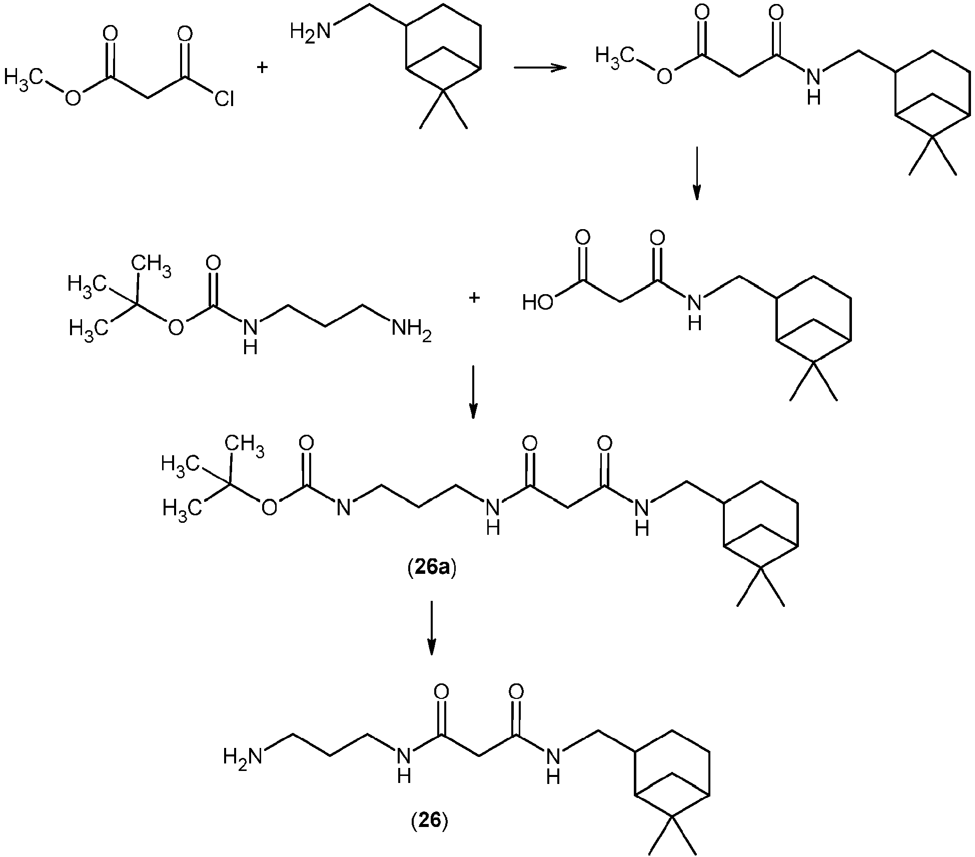

Die Synthese erfolgte analog zu Synthesebeispiel 16 aus Malonsäuremethylesterchlorid (250 µl, 2,33 mmol) und (-)-cis-Myrtanylamin (10-Pinanamin) (860 mg, 5,13 mmol) zum Malonsäuremethylester-N-(pinan-10-yl)amid, das zum Malonsäure-N-(pinan-10-yl)amid hydrolysiert wurde.

MS (ESI+) m/z (%): 240 (100, [M+H]⁺), 262 (55, [M+Na]⁺), 501 (10, [2M+Na]⁺). MS (ESI-) m/z (%): 238 (100, [M-H]⁻), 477 (100, [2M-H]⁻).

Dieses (295 mg, 1,327 mmol) wurde mit 3-Aminopropylcarbamidsäure-tert-butylester (N-Boc-1,3-propandiamin) (231 mg, 1,327 mmol) und TEA (371 µl, 2,654 mmol) in DMF (7 ml) mit 1,1 ml einer 1,6 M Lösung von T3P in Ethylacetat umgesetzt. Reinigung mittels Säulenchromatographie (Dichlormethan/Ethylacetat 50:50→Ethylacetat) ergab die Boc-geschützte Titelverbindung (26a) in Form eines farblosen Öls (278 mg).

¹H-NMR (CDCl₃): δ = 0,88 (d, 1H, J=8,5 Hz), 1,02 (s, 3H), 1,18 (s, 3H), 1,44 (s, 9H), 1,45-1,50 (m, 1H), 1,59-1,67 (m, 4H), 1,84-1,98 (m, 5H), 2,16-2,24 (m, 1H), 2,33-2,38 (m, 1H), 3,16 (s, 2H), 3,13-3,18 (m, 2H), 3,24-3,34 (m, 4H), 7,05 (s, 1H). MS (ESI+) m/z (%): 396 (100, [M+H]⁺), 296 (100, [M-Boc+H]⁺), 418 (100, [M+Na]+), 791(100, [2M+H]⁺), 813 (100, [2M+Na]⁺).

Das so erhaltene Produkt (0,703 mmol) wurde auf analoge Weise entschützt, wobei das Hydrochlorid der Titelverbindung (26) in Form eines weißen Feststoffs (260 mg) erhalten wurde.

¹H-NMR (DMSO-d₆): δ = 0,84 (d, 1H, J=8,5 Hz), 0,99 (s, 3H), 1,15 (s, 3H), 1,38-1,45 (m, 1H), 1,65-1,72 (m, 2H), 1,79-1,91 (m, 5H), 2,06-2,14 (m, 1H), 2,29-2,34 (m, 1H), 2,75-2,83 (m, 2H), 3,01 (s, 2H), 3,03-3,08 (m, 2H), 3,10-3,16 (m, 2H), 3,64-3,73 (m, 1H), 7,82 (brs, 2H). MS (ESI+) m/z (%): 296 (100, [M+H]⁺), 318 (90, [M+Na]⁺), 591 (100, [2M+H]⁺), 613 (100, [2M+Na]⁺).

### Svnthesebeispiel 20

### Herstellung von N-(3-Aminopropyl)-N'-tetrahydrochinolinmalonsäurediamid (108)

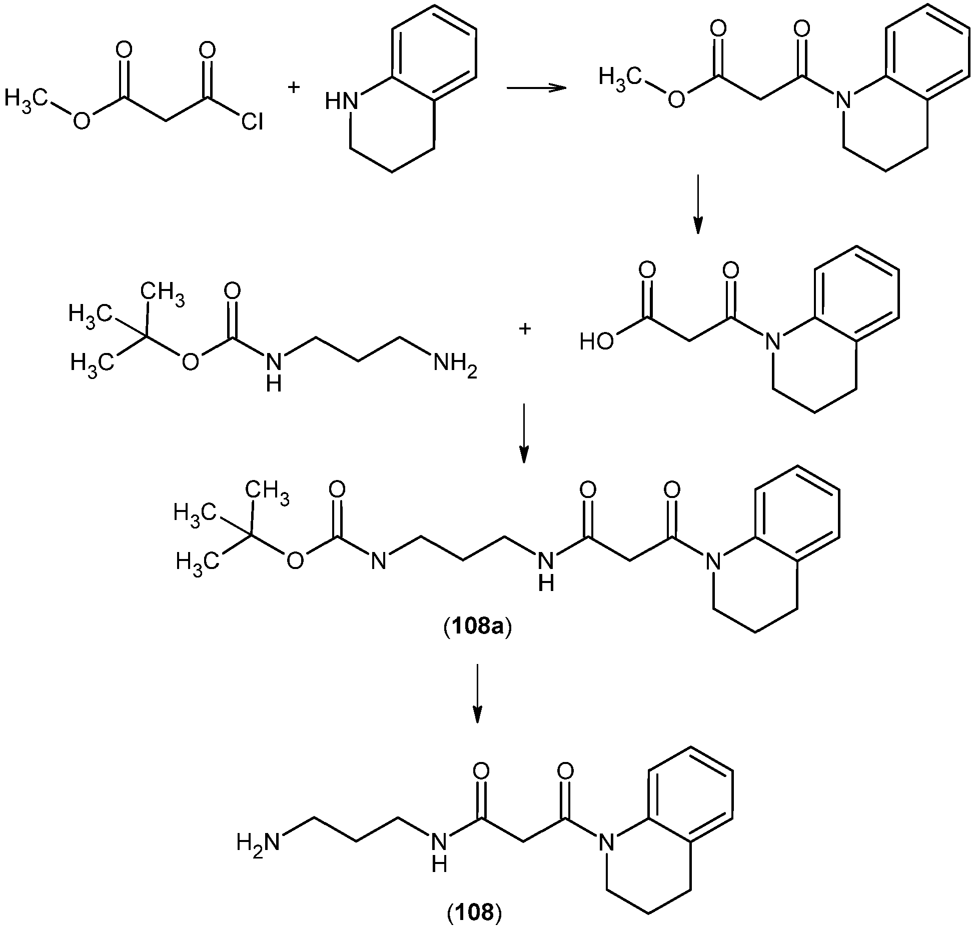

Die Synthese erfolgte analog zu Synthesebeispiel 17 aus Malonsäuremethylesterchlorid (175 µl, 1,63 mmol) und 1,2,3,4-Tetrahydrochinolin (478 mg, 3,59 mmol) zum Malonsäuremethylester-N-tetrahydrochinolinamid, das zum Malonsäure-N-tetrahydrochinolinamid hydrolysiert wurde.

MS (ESI+) m/z (%): 220 (100, [M+H]⁺), 242 (100, [M+Na]⁺), 461 (17, [2M+Na]⁺). MS (ESI-) m/z (%): 218 (100, [M-H]⁻).

Dieses (250 mg, 1,140 mmol) wurde mit 3-Aminopropylcarbamidsäure-tert-butylester (N-Boc-1,3-propandiamin) (199 mg, 1,140 mmol) und TEA (318 µl, 2,280 mmol) in DMF (10 ml) mit 900 µl einer 1,6 M Lösung von T3P in Ethylacetat umgesetzt. Reinigung mittels Säulenchromatographie (Dichlormethan/Ethylacetat 70:30→40:60) ergab die Boc-geschützte Titelverbindung (108a) in Form eines farblosen Öls (230 mg).

¹H-NMR (CDCl₃): δ = 1,43 (s, 9H), 1,67 (quint, 2H, J=6,7 Hz), 1,98 (quint, 2H, J=6,7 Hz), 2,72 (brs, 2H), 3,16 (q, 2H, J=6,2 Hz), 3,34 (q, 2H, J=6,4 Hz), 3,47 (s, 2H), 3,82 (t, 2H, J=6,7 Hz), 5,00 (brs, 1H), 7,09-7,23 (m, 4H). MS (ESI+) m/z (%): 376 (100, [M+H]+), 398 (100, [M+Na]⁺), 276 (100,[M-Boc+H]+), 773 (100, [2M+Na]⁺). MS (ESI-) m/z (%): 374 (100, [M-H]⁻).

Das so erhaltene Produkt (275 mg, 0,732 mmol) wurde auf analoge Weise entschützt, wobei das Hydrochlorid der Titelverbindung (108) in Form eines weißen Pulvers (264 mg) erhalten wurde.

¹H-NMR (DMSO-d₆): δ = 1,67 (quint, 2H, J=7,1 Hz), 1,87 (quint, 2H, J=6,6), 2,69 (t, 2H, J=6,6 Hz), 2,78 (q, 2H, J=6,6 Hz), 3,11 (q, 2H, 6,5 Hz), 3,67 (s, 2H), 3,64-3,72 (m, 2H), 7,09-7,20 (m, 4H), 7,82 (brs, 2H), 8,22 (brs, 1H). MS (ESI+) m/z (%): 276 (100, [M+H]+), 298 (8, [M+Na]⁺), 551 (11, [2M+H]⁺), 573 (18, [2M+Na]⁺).

### Beispiele 1 bis 26, Vergleichsbeispiele 1 bis 8 - Alzheimer-Testung

Die in den obigen Synthesebeispielen hergestellten bzw. käuflich erworbenen Substanzen (1) bis (26) und (101) bis (108) wurden in einem 96-Well-Verfahren in dem eingangs erwähnten Screening-Assay unter Verwendung des Fadenwurms *Caenorhabditis elegans* (Pretsch et al., s.o.) in einem standarisierten β-Amyloid-Testsystem unter Verwendung des transgenen *C. elegans-Stamms* CL2659 auf ihre potenzielle Eignung als Mittel zur Behandlung der Alzheimer-Krankheit getestet. Dazu wurden Wurmlarven im Stadium L3 in einer Dichte von 10-20 Würmern pro Well eingesetzt. Die Expression von β-Amyloid in den Muskelzellen wurde durch Temperaturerhöhung induziert und die damit einhergehende Paralyse bis zum Ende des Tests nach 48 h in regelmäßigen Zeitabständen erfasst. Bei diesem Modell sollte bei aktiven Substanzen in Konzentrationen, bei denen es zu einer Reduktion der β-Amyloid-Expression kommt, im Vergleich zu einer Kontrollsubstanz eine signifikante Verzögerung der durch neurotoxisch wirkendes β-Amyloid hervorgerufenen Paralyse der Larven zu beobachten sein.

Dazu wurden die obigen Substanzen in Konzentrationen von 1 mg/ml, 100 µg/ml und 10 µg/ml im Vergleich zu Quercetin als Negativkontrolle getestet. Die Testergebnisse sind in der nachstehenden Tabelle 1 angegeben, wobei für die erfindungsgemäßen Beispiele jener Konzentrationsbereich (in µg/ml), in dem eine Verzögerung der Paralyse feststellbar war, angegeben ist ("Alzheimer"), während für die Vergleichsbeispiele selbst in einer Konzentration von 1 mg/ml keine Wirkung beobachtbar war.

### Beispiele 27 bis 40, Vergleichsbeispiele 9 bis 16 - Parkinson-Testung

Dieselben Substanzen wie oben wurden in einem 96-Well-Verfahren in dem eingangs erwähnten Screening-Assay unter Verwendung des Fadenwurms *Caenorhabditis elegans* (Pretsch et al., s.o.) in einem standarisierten α-Synuclein-Testsystem unter Verwendung des transgenen *C. elegans-Stamms* NL5901 auf ihre potenzielle Eignung als Mittel zur Behandlung der Parkinson-Krankheit getestet. Dieser Wurmstamm exprimiert an grün fluoreszierendes Protein (GFP) gebundenes humanes α-Synuclein in allen Muskelzellen. Die GFP-Markierung erlaubt eine direkte Detektion der exprimierten Proteinmengen mittels eines Multiplattenreaders, die 24 bis 48 h lang durchgeführt wird. Bei diesem Testmodell sollte bei aktiven Substanzen in Konzentrationen, bei denen es zu einer Reduktion der α-Synuclein-Expression kommt, im Vergleich zu einer Kontrollsubstanz eine signifikante Verringerung der Expression von neurotoxischem α-Synuclein zu beobachten sein.

Dazu wurden die Substanzen erneut in Konzentrationen von 1 mg/ml, 100 µg/ml und 10 µg/ml, hier allerdings im Vergleich zu dem eingangs erwähnten Parkinson-Therapeutikum Levodopa als Kontrolle getestet. Die Testergebnisse sind ebenfalls in der umseitigen Tabelle 1 angegeben, wobei für die erfindungsgemäßen Beispiele wiederum jener Konzentrationsbereich (in µg/ml), in dem eine Verringerung der α-Synuclein-Expression feststellbar war, angegeben ist ("Parkinson"), während für die Vergleichsbeispiele auch in diesem Test selbst in einer Konzentration von 1 mg/ml keine Wirkung beobachtbar war.

**Tabelle 1**

| **Verb. Nr.** | **Chemischer Name** | **Alzheimer** | **Parkinson** | **Bewertung** |
|---|---|---|---|---|
| (1) | Malonsäure | > 100 | > 100 | + |
| (2) | Malonsäurediamid | > 10 | > 10 | ++ |
| (3) | Dinatriummalonat | > 10 | > 10 | ++ |
| (4) | Malonsäuredimethylester | > 10 | > 10 | ++ |
| (5) | Kaliummethylmalonat | > 10 | > 10 | ++ |
| (6) | Malonsäuremethylesteramid | > 100 | > 100 | + |
| (7) | N-(3-Aminopropyl)malonsäureamid | > 100 | > 100 | + |
| (8) | Malonsäureadamantylester | > 100 | > 100 | + |
| (9) | Malonsäureadamantan-1-ylethylester | > 10 | > 10 | ++ |
| (10) | Malonsäureadamantan-1-ylester-N-(3-aminopropyl)amid | > 10 | > 10 | ++ |
| (11) | Malonsäureadamantan-1-ylester-N-(3-aminopropyl)-N-methylamid | > 10 | > 10 | ++ |
| (12) | Malonsäureadamantan-1-ylesterpiperazinamid | > 10 | > 10 | ++ |
| (13) | Malonsäureadamantan-1-ylester-4-(4-aminobutyryl)piperazinamid | > 10 | > 10 | ++ |
| (14) | Malonsäureadamantan-1-ylester-4-aminopiperidinamid | > 10 | > 10 | ++ |
| (15) | Malonsäureadamantan-1-ylester-(4-(4-aminobutyryl)amino)piperidinamid | > 10 | > 10 | ++ |
| (16) | Malonsäuremethylester-N-(adamantan-1-yl)amid | > 10 | > 10 | ++ |
| (17) | N-(Adamantan-1-yl)malonsäureamid | > 10 | > 10 | ++ |
| (18) | N-Adamantan-1-yl-N'-(3-aminopropyl)malonsäurediamid | > 100 | > 100 | + |
| (19) | N-Adamantan-1-yl-N'-(3-aminopropyl)-N'-methylmalonsäurediamid | > 10 | > 10 | ++ |
| (20) | N-Adamantan-1-yl-N'-piperazinmalonsäurediamid | > 10 | > 10 | ++ |
| (21) | N-Adamantan-1-yl-N'-(4-(4-aminobutyryl)piperazin)malonsäurediamid | > 10 | > 10 | ++ |
| (22) | N-(3-Aminopropyl)-N'-morpholinmalonsäurediamid | > 10 | > 10 | ++ |
| (23) | N-(3-Aminopropyl)-N'-piperidinmalonsäurediamid | > 10 | > 10 | ++ |
| (24) | N-(3-Aminopropyl)-N'-decahydrochinolinmalonsäurediamid | > 10 | > 10 | ++ |
| (25) | N-(3-Aminopropyl)-N'-decahydroisochinolinmalonsäurediamid | > 10 | > 10 | ++ |
| (26) | N-(3-Aminopropyl)-N'-(pinan-10-yl)malonsäurediamid | > 10 | > 10 | ++ |
| (101) | Dimethylmalonsäure | - | - | - |
| (102) | Methylmalonsäuredimethylester | - | - | - |
| (103) | 1-Adamantancarbonsäure-N-(3-aminopropyl)amid | - | - | - |
| (104) | 3-Aminopropylcarbamidsäure-tert-butylester | - | - | - |
| (105) | 3-(Methylamino)propylcarbamidsäure-tert-butylester | - | - | - |
| (106) | 1-Adamantanol | - | - | - |
| (107) | 1-Adamantanamin | - | - | - |
| (108) | N-(3-Aminopropyl)-N'-tetrahydrochinolinmalonsäurediamid | - | - | - |

Die obigen Testergebnisse belegen eindeutig, dass Malonsäure sowie eine Vielzahl von Derivaten davon viel versprechende potenzielle Wirkstoffe sowohl gegen die Alzheimer- als auch gegen die Parkinson-Krankheit, möglicherweise aber auch gegen die Huntington-Krankheit oder Prion-Erkrankungen darstellen.

Die für die verschiedenen Substitutionsmuster erzielten Ergebnisse zeigen, dass in den wirksamen Molekülen sowohl freie Carboxylgruppen als auch Salze, Ester und substituierte oder unsubstituierte Amide davon enthalten sein können, ohne die Wirksamkeit zu beeinträchtigen. Auch die Sperrigkeit der Substituenten scheint keine signifikante Rolle zu spielen, da sowohl kleine (Methyl, Ethyl) als auch voluminöse (Adamantyl) Gruppen vergleichbare Wirksamkeit zeigten.

Wie auch die Vergleichsbeispiele belegen, beruht diese Wirksamkeit offensichtlich insbesondere auf der Gegenwart einer unsubstituierten Malonsäure-Gruppierung, da weder Dimethylmalonsäure (101) noch Methylmalonsäuredimethylester (102) sich als wirksam erwiesen haben. Wie die Unwirksamkeit der Verbindungen (103) bis (106) beweist, beeinflussen auch die in den erfindungsgemäß wirksamen Derivaten von Malonsäure enthaltenen Substituenten wie etwa die Adamantyl- oder die AminopropylGruppe das Ausmaß der Wirksamkeit kaum. Die Unwirksamkeit des Tetrahydrochinolinamids (108) ist hingegen eindeutig auf die Gegenwart des aromatischen Substituenten zurückzuführen, zumal das Decahydrochinolinamid (24) genau wie dessen Isomer, das Decahydroisochinolinamid (25), bereits in einer Konzentration > 10 µg/ml wirksam waren. Aus diesem Grund sind Derivate mit ungesättigten und insbesondere aromatischen Substituenten wohl als ungeeignet anzusehen und daher aus dem Schutzumfang der Erfindung ausgenommen.

Dazu kommt, dass der in den Testreihen gewählte Verdünnungsfaktor von 1:10 einen breiten Spielraum für die tatsächlich wirksamen Konzentrationen lässt. Dieser schließt ja beispielsweise nicht aus, dass eine bereits in einer Konzentration > 10 µg/ml wirksame und daher mit "++" bewertete Verbindung möglicherweise erst in einer knapp unter dem Grenzwert von 100 µg/ml liegenden Konzentration, z.B. ab 90 µg/ml, zur Wirkung kommt, während die Wirksamkeitsgrenze einer erst über 100 µg/ml wirksamen und folglich mit "+" bewerteten Verbindung vielleicht nur knapp über dem Grenzwert liegt. Solche Verbindungen können sich tatsächlich auch nur um wenige Prozentpunkte in ihrer Wirksamkeit unterscheiden.

Weiters ist zu bedenken, dass die in den Tests tatsächlich verfügbare Menge der jeweiligen Verbindung, d.h. die molare Menge, natürlich vom Molekulargewicht abhängt, das jedoch in weiten Grenzen variiert. So weisen etwa freie Malonsäure (1) (104,06 g/mol) und Malonsäurediamid (2) (102,09 g/mol) nur etwas mehr als ein Viertel des Molekulargewichts von Malonsäureadamantan-1-ylester-4-(4-aminobutyryl)piperazin-amid (13) (391,51 g/mol) oder Malonsäureadamantan-1-ylester-(4-(4-aminobutyryl)-amino)piperidinamid (15) (405,54 g/mol) auf.

Weitere Untersuchungen hinsichtlich der Optimierung der Wirksamkeit mittels weiterer Variationen des Substitutionsmusters und der Verdünnungsfaktoren in den Tests sind daher derzeit Gegenstand der Forschungen der Erfinder.

## Patentansprüche

1. Malonsäure und Derivate davon der nachstehenden Formel (I) zur Verwendung als Arzneimittel zur Wiederherstellung der Metallhomöostase bei einem Patienten: 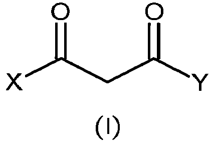 worin X und Y jeweils unabhängig aus -O⁻M⁺, -OR₁ und -NR₂R₃ ausgewählt sind, worin M⁺ aus ein- oder mehrwertigen Metallanionen ausgewählt ist und R₁, R₂ und R₃ jeweils unabhängig aus Wasserstoff und gesättigten, unverzweigten, verzweigten oder zyklischen Kohlenwasserstoff-Resten mit 1 bis 25 Kohlenstoffatomen ausgewählt sind, worin ein oder mehrere Kohlenstoffatome gegebenenfalls durch O oder N ersetzt sind, wobei R₂ und R₃ gegebenenfalls miteinander verbunden sind und zusammen mit dem Stickstoffatom einen heterozyklischen Kohlenwasserstoff-Rest bilden.

2. Verbindung zur Verwendung nach Anspruch 1, **dadurch gekennzeichnet, dass** in Formel (I) sowohl X als auch Y für -O⁻M⁺ stehen, worin M⁺ jeweils unabhängig aus ein- und mehrwertigen Metallanionen ausgewählt ist, wobei M⁺ vorzugsweise für ein Alkalimetall- oder Erdalkalimetallion, noch bevorzugter für ein Alkalimetallion, steht.

3. Verbindung zur Verwendung nach Anspruch 1 oder 2, **dadurch gekennzeichnet, dass** R₁, R₂ und R₃ jeweils unabhängig aus Wasserstoff und gesättigten, unverzweigten, verzweigten oder zyklischen Kohlenwasserstoff-Resten mit 1 bis 15 Kohlenstoffatomen ausgewählt sind.

4. Verbindung zur Verwendung nach Anspruch 3, **dadurch gekennzeichnet, dass** R₁, R₂ und R₃ jeweils unabhängig aus Wasserstoff und Kohlenwasserstoff-Resten mit 1 bis 10 Kohlenstoffatomen ausgewählt sind.

5. Verbindung zur Verwendung nach Anspruch 4, **dadurch gekennzeichnet, dass** R₁, R₂ und R₃ jeweils unabhängig aus Wasserstoff, Methyl, Ethyl, Adamantyl und 3-Aminopropyl ausgewählt sind.

6. Verbindung zur Verwendung nach einem der Ansprüche 1 bis 5, **dadurch gekennzeichnet, dass** die Verbindung aus den folgenden ausgewählt ist: Malonsäure, Malonsäurediamid, Dinatriummalonat, Malonsäuredimethylester, Malonsäuremethylester-Kaliumsalz, Malonsäuremethylesteramid, N-(3-Aminopropyl)malonsäureamid, Malonsäureadamantan-1-ylester, Malonsäureadamantan-1-ylethylester, Malonsäureadamantan-1-ylester-N-(3-aminopropyl)amid, Malonsäureadamantan-1-ylester-N-(3-aminopropyl)-N-methylamid, Malonsäureadamantan-1-ylesterpiperazinamid, Malonsäureadamantan-1-ylester-4-(4-aminobutyryl)piperazinamid, Malonsäureadamantan-1-ylester-4-aminopiperidinamid, Malonsäureadamantan-1-ylester-(4-(4-aminobutyryl)-amino)piperidinamid, Malonsäuremethylester-N-(adamantan-1-yl)amid, N-(Adamantan-1-yl)malonsäureamid, N-Adamantan-1-yl-N'-(3-aminopropyl)malonsäurediamid, N-Adamantan-1-yl-N'-(3-aminopropyl)-N'-methylmalonsäurediamid, N-Adamantan-1-yl-N'-piperazinmalonsäurediamid, N-Adamantan-1-yl-N'-(4-(4-aminobutyryl)piperazin)malonsäurediamid, N-(3-Aminopropyl)-N'-morpholinmalonsäurediamid, N-(3-Aminopropyl)-N'-piperidinmalonsäurediamid, N-(3-Aminopropyl)-N'-decahydrochinolin-malonsäurediamid, N-(3-Aminopropyl)-N'-decahydroisochinolinmalonsäurediamid und N-(3-Aminopropyl)-N'-(6,6-dimethylbicyclo[3.1 .1]heptan-2-ylmethyl)malonsäurediamid.

7. Verbindung zur Verwendung nach einem der Ansprüche 1 bis 6, **dadurch gekennzeichnet, dass** die Wiederherstellung der Metallhomöostase zur Behandlung einer neurodegenerativen Erkrankung dient.

8. Verbindung zur Verwendung nach Anspruch 7, **dadurch gekennzeichnet, dass** die neurodegenerative Erkrankung aus der Alzheimer-Krankheit, der Parkinson-Krankheit und Chorea Huntington ausgewählt ist.

9. Verbindung zur Verwendung nach Anspruch 8, **dadurch gekennzeichnet, dass** die neurodegenerative Erkrankung die Alzheimer- oder die Parkinson-Krankheit ist.

10. Pharmazeutische Zusammensetzung zur Wiederherstellung der Metallhomöostase bei einem Patienten, die eine Verbindung wie in einem der Ansprüche 1 bis 6 definiert, zumindest einen pharmazeutisch annehmbaren Exzipienten umfasst und gegebenenfalls einen oder mehrere weitere pharmazeutisch annehmbare Inhaltsstoffe umfasst.

11. Pharmazeutische Zusammensetzung nach Anspruch 10, **dadurch gekennzeichnet, dass** sie zur Behandlung einer neurodegenerativen Erkrankung dient.

12. Pharmazeutische Zusammensetzung nach Anspruch 11, **dadurch gekennzeichnet, dass** sie zur Behandlung der Alzheimer- oder der Parkinson-Krankheit dient.
